# EUROPEAN PATENT APPLICATION

(11) **EP 4 458 852 A1**
(43) Date of publication of application: **06.11.2024**
(21) Application number: 22914995.0
(22) Date of filing: 29.12.2022
(51) Int. Cl.: C07K 16/28, A61K 39/00, A61K 39/395, A61P 3/00, A61P 3/04, A61P 3/06, A61P 3/08, A61P 9/00

(54) **ANTI-?KLOTHO ANTIBODY AND USE THEREOF**

(30) Priority: 30.12.2021 CN 202111683753
(71) Applicant: Shanghai JMT-Bio Technology Co., Ltd., Shanghai 200032 (CN)
(72) Inventor: FENG, Xu, Shanghai 200032 (CN); SONG, Liping, Shanghai 200032 (CN); FAN, Yi, Shanghai 200032 (CN); MENG, Yali, Shanghai 200032 (CN)
(74) Representative: Gill Jennings & Every LLP
(86) International application number: PCT/CN2022/143127
(87) International publication number: WO 2023/125745

(57) **Abstract**

Provided are an anti-βKlotho antibody and the use thereof. The anti-βBKlotho antibody can be used for treating βK-lotho-mediated or βKlotho-FGFR1c-FGF21-mediated diseases, especially abnormal metabolic diseases, such as type 1 diabetes, type 2 diabetes, dyslipidemia, non-alcoholic steatohepatitis (NASH), non-alcoholic fatty liver disease (NAFLD), cardiovascular disease, metabolic syndrome or obesity.

## Description

### CROSS REFERECE OF RELATED APPLICATION

The present application claims priority to Chinese Patent Application No. 202111683753.3 filed on December 30, 2021, which is hereby incorporated by reference in its entirety for all purposes.

### TECHNICAL FIELD

The present application generally relates to the biopharmaceutical field, and in particular, to novel anti-βKlotho antibodies or antigen-binding fragments thereof, pharmaceutical compositions comprising the antibodies or antigen-binding fragments thereof, and medical and pharmaceutical uses of the antibodies or antigen-binding fragments thereof.

### BACKGROUND

Fibroblast growth factor 21 (FGF21), a member of the FGF19 subfamily of the fibroblast growth factor family, plays an important role in regulating glucose, lipid and energy metabolism. In addition to FGF21, the FGF19 subfamily has two other members, FGF19 and FGF23. Unlike classical fibroblasts, which require heparin binding to exert their function, the three proteins of the FGF19 subfamily do not bind to heparin, and can be secreted into the blood to function physiologically in other tissues or organs of the body. Thus, the three proteins including FGF21 are also called endocrine fibroblast growth factors. FGF21 is mainly expressed in liver, but also minorly in pancreas and brain. It can act on adipose tissues, the central nervous system and other tissues and organs, so as to improve insulin sensitivity, increase glucose clearance in the blood, inhibit hepatic glycogenolysis, increase energy consumption, enhance hepatic ketogenesis, reduce triglyceride level in the blood and so on. In addition to FGF21, FGF19 is mainly expressed in small intestines, and mainly acts on hepatocytes and plays a role in regulating bile acid synthesis and secretion in hepatocytes. FGF23 is a phosphaturic hormone that plays a vital role in regulating phosphate balance in humans.

The three proteins in the FGF19 subfamily, including FGF21, exert their physiological functions by activating the FGF receptor (FGFR). They cannot activate the FGF receptor by themselves, but need to exert their physiological functions with the help of co-receptors, i.e., the Klotho proteins. The Klotho proteins are divided into αKlotho and βKlotho, which are necessary for high-affinity binding of FGF19, FGF21, and FGF23 to their homologous FGF receptors. αKlotho mediates the activation of the FGF receptor by FGF23, while βklotho mediates the receptor activation by FGF19 and FGF21. βklotho is a single-pass type I membrane protein with an extracellular domain consisting of two internal repeats homologous to members of family 1 glycosidases but lacking glucosidase catalytic activity. βklotho expression was detected in liver, pancreas, fat, and the central nervous system. Studies showed that CYP7A1 and CY8B1 mRNA levels in βklotho-deficient (KLB^{-/-}) mice were elevated, and bile acid synthesis and secretion were increased.

FGF21 binds to the FGF receptor and βKlotho to form a ternary complex, which promotes the dimerization of the FGF receptor, and then a six-membered complex is formed to activate the FGF receptor. The activated FGF receptor will further activate the downstream MAPK(Erk1/2) and AKT1 signaling pathways to exert its biological functions. FGF21 activates the FGF receptor on adipocytes, so as to promote the expression of GLUT-1 in adipocytes and increase the absorption of glucose by adipocytes. In addition, FGF21 can upregulate the expression of UCP-1 and promote the brown-like transformation of adipocytes. FGF21 can also induce the expression and secretion of adiponectin or other adipocytokines that enhance insulin sensitivity. Adiponectin and other adipokines act on other tissues or organs (e.g., the liver) to improve systemic metabolism. Recent studies also showed that FGF21 can act on the central nervous system, and in addition to indirectly regulating sugar, fat and energy metabolism, it also affects the appetite for alcohol and sugar in human or mouse. Transgenic mice overexpressing FGF21 exhibited a range of metabolic-related phenotypes, including slow growth, low blood glucose and triglyceride levels, resistance to type 2 diabetes associated with aging, islet hyperplasia, and obesity. In rat or non-human primate models, the animals, after injection with an FGF21 fusion protein, exhibited blood glucose level restoration, decreaseed triglycerides and cholesterol blood levels, increased glucose tolerance and increased insulin sensitivity. Furthermore, FGF21 increased the activity, metabolic rate and energy consumption of the animals, thus reducing the body weight and body fat. In a mouse model of non-alcoholic steatohepatitis, the modified FGF21 protein alleviated the degree of hepatic steatosis, reduced liver damage, and reduced molecular markers of inflammation in the liver. These studies suggest that FGF21 has potential for use in the treatment of metabolic-related diseases such as non-alcoholic steatohepatitis, type 2 diabetes, obesity, and dyslipidemia.

Among FGF receptors, only FGFR1c, 2c, 3c, and 4 can bind to Klotho, while FGFR1b, 2b, and 3b cannot bind to Klotho. *In vitro* experiments show that FGF21 can activate FGFR1c, 2c and 3c receptors with the help of βKlotho. *In vivo* experiments show that the effect of FGF21 on metabolism is mainly mediated by FGFR1c and βKlotho receptors. It is thus speculated that molecules mimicking the action of FGF21 and activating the FGFR1c and βklotho receptors (such as activating anti-βKlotho antibodies) are also potentially useful in in the treatment of metabolic-related diseases such as non-alcoholic steatohepatitis.

Currently, there are few studies on antibodies targeting βKlotho. Those with relatively rapid progresses includes BFKB8488A from Genentech (anti-FGFR1/KLB bispecific antibody) which is tested in clinical phase II; and NGM 313 from NGM Biopharmaceuticals (monoclonal agonist antibody) which is also tested in clinical phase II with clinical trial indications being non-alcoholic steatohepatitis. Products from other companies, such as Amgen, Regeneron and Novartis, are tested in the preclinical phase. Given that these drugs are in the early clinical stages and their efficacies are not yet definite, there is a demand for diversified treatment of antibody drugs targeting the above targets.

### SUMMARY OF THE INVENTION

In a first aspect, there is provided in the present application an anti-βKlotho antibody or an antigen-binding portion thereof, comprising a heavy chain variable region and a light chain variable region, wherein the heavy chain variable region comprises a heavy chain CDR1 (HCDR1), a heavy chain CDR2 (HCDR2) and a heavy chain CDR3 (HCDR3), and the light chain variable region comprises a light chain CDR1 (LCDR1), a light chain CDR2 (LCDR2) and a light chain CDR3 (LCDR3), and wherein:
(1) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.2 or has at least 80%, 85%, 90%, 95% or 99% identity to the sequence as set forth in SEQ ID NO.2, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.5 or has at least 80%, 85%, 90%, 95% or 99% identity to the sequence as set forth in SEQ ID NO.5, and the sequence of the LCDR3 is the sequence as set forth in SEQ ID NO.6 or has at least 80%, 85%, 90%, 95% or 99% identity to the sequence as set forth in SEQ ID NO.6; or
(2) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.14 or has at least 80%, 85%, 90%, 95% or 99% identity to the sequence as set forth in SEQ ID NO.14, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.15 or has at least 80%, 85%, 90%, 95% or 99% identity to the sequence as set forth in SEQ ID NO.15, and the sequence of the LCDR3 is the sequence as set forth in SEQ ID NO.6 or has at least 80%, 85%, 90%, 95% or 99% identity to the sequence as set forth in SEQ ID NO.6; or
(3) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.20 or has at least 80%, 85%, 90%, 95% or 99% identity to the sequence as set forth in SEQ ID NO.20, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.21 or has at least 80%, 85%, 90%, 95% or 99% identity to the sequence as set forth in SEQ ID NO.21, and the sequence of the LCDR3 is the sequence as set forth in SEQ ID NO.6 or has at least 80%, 85%, 90%, 95% or 99% identity to the sequence as set forth in SEQ ID NO.6; or
(4) the sequence of the HCDR1 is the sequence as set forth in SEQ ID NO.26 or has at least 80%, 85%, 90%, 95% or 99% identity to the sequence as set forth in SEQ ID NO.26, the sequence of the HCDR2 is the sequence as set forth in SEQ ID NO. 14 or has at least 80%, 85%, 90%, 95% or 99% identity to the sequence as set forth in SEQ ID NO. 14, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.27 or has at least 80%, 85%, 90%, 95% or 99% identity to the sequence as set forth in SEQ ID NO.27, and the sequence of the LCDR3 is the sequence as set forth in SEQ ID NO.6 or has at least 80%, 85%, 90%, 95% or 99% identity to the sequence as set forth in SEQ ID NO.6; or
(5) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.2 or has at least 80%, 85%, 90%, 95% or 99% identity to the sequence as set forth in SEQ ID NO.2, the sequence of the HCDR3 is the sequence as set forth in SEQ ID NO.31 or has at least 80%, 85%, 90%, 95% or 99% identity to the sequence as set forth in SEQ ID NO.31, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.5 or has at least 80%, 85%, 90%, 95% or 99% identity to the sequence as set forth in SEQ ID NO.5, and the sequence of the LCDR3 is the sequence as set forth in SEQ ID NO.6 or has at least 80%, 85%, 90%, 95% or 99% identity to the sequence as set forth in SEQ ID NO.6; or
(6) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO. 14 or has at least 80%, 85%, 90%, 95% or 99% identity to the sequence as set forth in SEQ ID NO. 14, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.34 or has at least 80%, 85%, 90%, 95% or 99% identity to the sequence as set forth in SEQ ID NO.34, and the sequence of the LCDR3 is the sequence as set forth in SEQ ID NO.6 or has at least 80%, 85%, 90%, 95% or 99% identity to the sequence as set forth in SEQ ID NO.6; or
(7) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.27 or has at least 80%, 85%, 90%, 95% or 99% identity to the sequence as set forth in SEQ ID NO.27, and the sequence of the LCDR3 is the sequence as set forth in SEQ ID NO.6 or has at least 80%, 85%, 90%, 95% or 99% identity to the sequence as set forth in SEQ ID NO.6; or
(8) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.42 or has at least 80%, 85%, 90%, 95% or 99% identity to the sequence as set forth in SEQ ID NO.42, and the sequence of the LCDR3 is the sequence as set forth in SEQ ID NO.6 or has at least 80%, 85%, 90%, 95% or 99% identity to the sequence as set forth in SEQ ID NO.6; or
(9) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.20 or has at least 80%, 85%, 90%, 95% or 99% identity to the sequence as set forth in SEQ ID NO.20, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.45 or has at least 80%, 85%, 90%, 95% or 99% identity to the sequence as set forth in SEQ ID NO.45, and the sequence of the LCDR3 is the sequence as set forth in SEQ ID NO.6 or has at least 80%, 85%, 90%, 95% or 99% identity to the sequence as set forth in SEQ ID NO.6; or
(10) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, the sequence of the HCDR3 is the sequence as set forth in SEQ ID NO.31 or has at least 80%, 85%, 90%, 95% or 99% identity to the sequence as set forth in SEQ ID NO.31, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.42 or has at least 80%, 85%, 90%, 95% or 99% identity to the sequence as set forth in SEQ ID NO.42, and the sequence of the LCDR3 is the sequence as set forth in SEQ ID NO.6 or has at least 80%, 85%, 90%, 95% or 99% identity to the sequence as set forth in SEQ ID NO.6;
wherein the sequences of the HCDRs and LCDRs are defined according to the Kabat definition.

In a second aspect, there is provided in the present application an anti-βKlotho antibody or an antigen-binding portion thereof, comprising a heavy chain variable region and a light chain variable region, wherein the heavy chain variable region comprises a heavy chain CDR1 (HCDR1), a heavy chain CDR2 (HCDR2) and a heavy chain CDR3 (HCDR3), and the light chain variable region comprises a light chain CDR1 (LCDR1), a light chain CDR2 (LCDR2) and a light chain CDR3 (LCDR3), and wherein:
(1) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.8 or has at least 80%, 85%, 90%, 95% or 99% identity to the sequence as set forth in SEQ ID NO.8, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO.10 or has at least 80%, 85%, 90%, 95% or 99% identity to the sequence as set forth in SEQ ID NO.10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(2) the sequence of the HCDR1 is the sequence as set forth in SEQ ID NO. 16 or has at least 80%, 85%, 90%, 95% or 99% identity to the sequence as set forth in SEQ ID NO. 16, the sequence of the HCDR2 is the sequence as set forth in SEQ ID NO. 17 or has at least 80%, 85%, 90%, 95% or 99% identity to the sequence as set forth in SEQ ID NO. 17, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(3) the sequence of the HCDR1 is the sequence as set forth in SEQ ID NO.22 or has at least 80%, 85%, 90%, 95% or 99% identity to the sequence as set forth in SEQ ID NO.22, the sequence of the HCDR2 is the sequence as set forth in SEQ ID NO.23 or has at least 80%, 85%, 90%, 95% or 99% identity to the sequence as set forth in SEQ ID NO.23, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(4) the sequence of the HCDR1 is the sequence as set forth in SEQ ID NO.28 or has at least 80%, 85%, 90%, 95% or 99% identity to the sequence as set forth in SEQ ID NO.28, the sequence of the HCDR2 is the sequence as set forth in SEQ ID NO. 17 or has at least 80%, 85%, 90%, 95% or 99% identity to the sequence as set forth in SEQ ID NO. 17, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(5) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.8 or has at least 80%, 85%, 90%, 95% or 99% identity to the sequence as set forth in SEQ ID NO.8, the sequence of the HCDR3 is the sequence as set forth in SEQ ID NO.32 or has at least 80%, 85%, 90%, 95% or 99% identity to the sequence as set forth in SEQ ID NO. 32, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(6) the sequence of the HCDR1 is the sequence as set forth in SEQ ID NO.35 or has at least 80%, 85%, 90%, 95% or 99% identity to the sequence as set forth in SEQ ID NO.35, the sequence of the HCDR2 is the sequence as set forth in SEQ ID NO. 17 or has at least 80%, 85%, 90%, 95% or 99% identity to the sequence as set forth in SEQ ID NO. 17, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(7) the sequence of the HCDR1 is the sequence as set forth in SEQ ID NO.39 or has at least 80%, 85%, 90%, 95% or 99% identity to the sequence as set forth in SEQ ID NO.39, the sequence of the HCDR2 is the sequence as set forth in SEQ ID NO.40 or has at least 80%, 85%, 90%, 95% or 99% identity to the sequence as set forth in SEQ ID NO.40, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(8) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.40 or has at least 80%, 85%, 90%, 95% or 99% identity to the sequence as set forth in SEQ ID NO.40, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(9) the sequence of the HCDR1 is the sequence as set forth in SEQ ID NO.46 or has at least 80%, 85%, 90%, 95% or 99% identity to the sequence as set forth in SEQ ID NO.46, the sequence of the HCDR2 is the sequence as set forth in SEQ ID NO.23 or has at least 80%, 85%, 90%, 95% or 99% identity to the sequence as set forth in SEQ ID NO.23, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(10) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.40 or has at least 80%, 85%, 90%, 95% or 99% identity to the sequence as set forth in SEQ ID NO.40, the sequence of the HCDR3 is the sequence as set forth in SEQ ID NO.32 or has at least 80%, 85%, 90%, 95% or 99% identity to the sequence as set forth in SEQ ID NO.32, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO. 11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6;
wherein the sequences of the HCDRs and LCDRs are defined according to the IMGT definition.

In a third aspect, there is provided in the present application an anti-βKlotho antibody or an antigen-binding portion thereof comprising a heavy chain variable region and a light chain variable region, wherein:
the heavy chain variable region has the amino acid sequence as set forth in SEQ ID NO.12, 18, 24, 29, 33, 36, 41, 43, 47 or 49, or has an amino acid sequence having at least 80%, at least 85%, at least 90%, at least 95% or at least 99% identity to the amino acid sequence as set forth in SEQ ID NO.12, 18, 24, 29, 33, 36, 41, 43, 47 or 49; and/or
the light chain variable region has the amino acid sequence as set forth in SEQ ID NO.13, 19, 25, 30, 37, 44 or 48, or has an amino acid sequence having at least 80%, at least 85%, at least 90%, at least 95% or at least 99% identity to the amino acid sequence as set forth in SEQ ID NO.13, 19, 25, 30, 37, 44 or 48.

In a fourth aspect, there is provided in the present application an anti-βKlotho antibody or an antigen-binding portion thereof comprising a heavy chain variable region comprising a heavy chain CDR1 (HCDR1), a heavy chain CDR2 (HCDR2), and a heavy chain CDR3 (HCDR3), wherein:
(1) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.2 or has at least 80%, 85%, 90%, 95% or 99% identity to the sequence as set forth in SEQ ID NO.2, and the sequence of the HCDR3 is the sequence as set forth in SEQ ID NO.3 or has at least 80%, 85%, 90%, 95% or 99% identity to the sequence as set forth in SEQ ID NO.3; or
(2) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.14 or has at least 80%, 85%, 90%, 95% or 99% identity to the sequence as set forth in SEQ ID NO.14, and the sequence of the HCDR3 is the sequence as set forth in SEQ ID NO.3 or has at least 80%, 85%, 90%, 95% or 99% identity to the sequence as set forth in SEQ ID NO.3; or
(3) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.20 or has at least 80%, 85%, 90%, 95% or 99% identity to the sequence as set forth in SEQ ID NO. 20, and the sequence of the HCDR3 is the sequence as set forth in SEQ ID NO.3 or has at least 80%, 85%, 90%, 95% or 99% identity to the sequence as set forth in SEQ ID NO.3; or
(4) the sequence of the HCDR1 is the sequence as set forth in SEQ ID NO.26 or has at least 80%, 85%, 90%, 95% or 99% identity to the sequence as set forth in SEQ ID NO.26, the sequence of the HCDR2 is the sequence as set forth in SEQ ID NO.14 or has at least 80%, 85%, 90%, 95% or 99% identity to the sequence as set forth in SEQ ID NO.14, and the sequence of the HCDR3 is the sequence as set forth in SEQ ID NO.3 or has at least 80%, 85%, 90%, 95% or 99% identity to the sequence as set forth in SEQ ID NO.3; or
(5) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.2 or has at least 80%, 85%, 90%, 95% or 99% identity to the sequence as set forth in SEQ ID NO.2, and the sequence of the HCDR3 is the sequence as set forth in SEQ ID NO.31 or has at least 80%, 85%, 90%, 95% or 99% identity to the sequence as set forth in SEQ ID NO.31; or
(6) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, and the sequence of the HCDR3 is the sequence as set forth in SEQ ID NO.3 or has at least 80%, 85%, 90%, 95% or 99% identity to the sequence as set forth in SEQ ID NO.3; or
(7) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, and the sequence of the HCDR3 is the sequence as set forth in SEQ ID NO.31 or has at least 80%, 85%, 90%, 95% or 99% identity to the sequence as set forth in SEQ ID NO.31;
wherein the sequences of the HCDRs are defined according to the Kabat definition.

In a fifth aspect, there is provided in the present application an anti-βKlotho antibody or an antigen-binding portion thereof comprising a light chain variable region comprising a light chain CDR1 (LCDR1), a light chain CDR2 (LCDR2), and a light chain CDR3 (LCDR3), wherein:
(1) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.5 or has at least 80%, 85%, 90%, 95% or 99% identity to the sequence as set forth in SEQ ID NO.5, and the sequence of the LCDR3 is the sequence as set forth in SEQ ID NO.6 or has at least 80%, 85%, 90%, 95% or 99% identity to the sequence as set forth in SEQ ID NO.6; or
(2) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.15 or has at least 80%, 85%, 90%, 95% or 99% identity to the sequence as set forth in SEQ ID NO.15, and the sequence of the LCDR3 is the sequence as set forth in SEQ ID NO.6 or has at least 80%, 85%, 90%, 95% or 99% identity to the sequence as set forth in SEQ ID NO.6; or
(3) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.21 or has at least 80%, 85%, 90%, 95% or 99% identity to the sequence as set forth in SEQ ID NO.21, and the sequence of the LCDR3 is the sequence as set forth in SEQ ID NO.6 or has at least 80%, 85%, 90%, 95% or 99% identity to the sequence as set forth in SEQ ID NO.6; or
(4) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.27 or has at least 80%, 85%, 90%, 95% or 99% identity to the sequence as set forth in SEQ ID NO.27, and the sequence of the LCDR3 is the sequence as set forth in SEQ ID NO.6 or has at least 80%, 85%, 90%, 95% or 99% identity to the sequence as set forth in SEQ ID NO.6; or
(5) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.34 or has at least 80%, 85%, 90%, 95% or 99% identity to the sequence as set forth in SEQ ID NO.34, and the sequence of the LCDR3 is the sequence as set forth in SEQ ID NO.6 or has at least 80%, 85%, 90%, 95% or 99% identity to the sequence as set forth in SEQ ID NO.6; or
(6) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.42 or has at least 80%, 85%, 90%, 95% or 99% identity to the sequence as set forth in SEQ ID NO.42, and the sequence of the LCDR3 is the sequence as set forth in SEQ ID NO.6 or has at least 80%, 85%, 90%, 95% or 99% identity to the sequence as set forth in SEQ ID NO.6; or
(7) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.45 or has at least 80%, 85%, 90%, 95% or 99% identity to the sequence as set forth in SEQ ID NO.45, and the sequence of the LCDR3 is the sequence as set forth in SEQ ID NO.6 or has at least 80%, 85%, 90%, 95% or 99% identity to the sequence as set forth in SEQ ID NO.6;
wherein the sequences of the LCDRs are defined according to the Kabat definition.

In a sixth aspect, there is provided in the present application an anti-βKlotho antibody or an antigen-binding portion thereof comprising a heavy chain variable region comprising a heavy chain CDR1 (HCDR1), a heavy chain CDR2 (HCDR2) and a heavy chain CDR3 (HCDR3), wherein:
(1) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.8 or has at least 80%, 85%, 90%, 95% or 99% identity to the sequence as set forth in SEQ ID NO.8, and the sequence of the HCDR3 is the sequence as set forth in SEQ ID NO.9 or has at least 80%, 85%, 90%, 95% or 99% identity to the sequence as set forth in SEQ ID NO.9; or
(2) the sequence of the HCDR1 is the sequence as set forth in SEQ ID NO. 16 or has at least 80%, 85%, 90%, 95% or 99% identity to the sequence as set forth in SEQ ID NO. 16, the sequence of the HCDR2 is the sequence as set forth in SEQ ID NO. 17 or has at least 80%, 85%, 90%, 95% or 99% identity to the sequence as set forth in SEQ ID NO. 17, and the sequence of the HCDR3 is the sequence as set forth in SEQ ID NO.9 or has at least 80%, 85%, 90%, 95% or 99% identity to the sequence as set forth in SEQ ID NO.9; or
(3) the sequence of the HCDR1 is the sequence as set forth in SEQ ID NO.22 or has at least 80%, 85%, 90%, 95% or 99% identity to the sequence as set forth in SEQ ID NO.22, the sequence of the HCDR2 is the sequence as set forth in SEQ ID NO.23 or has at least 80%, 85%, 90%, 95% or 99% identity to the sequence as set forth in SEQ ID NO. 23, and the sequence of the HCDR3 is the sequence as set forth in SEQ ID NO.9 or has at least 80%, 85%, 90%, 95% or 99% identity to the sequence as set forth in SEQ ID NO.9; or
(4) the sequence of the HCDR1 is the sequence as set forth in SEQ ID NO.28 or has at least 80%, 85%, 90%, 95% or 99% identity to the sequence as set forth in SEQ ID NO.28, the sequence of the HCDR2 is the sequence as set forth in SEQ ID NO. 17 or has at least 80%, 85%, 90%, 95% or 99% identity to the sequence as set forth in SEQ ID NO. 17, and the sequence of the HCDR3 is the sequence as set forth in SEQ ID NO.9 or has at least 80%, 85%, 90%, 95% or 99% identity to the sequence as set forth in SEQ ID NO.9; or
(5) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.8 or has at least 80%, 85%, 90%, 95% or 99% identity to the sequence as set forth in SEQ ID NO.8, and the sequence of the HCDR3 is the sequence as set forth in SEQ ID NO.32 or has at least 80%, 85%, 90%, 95% or 99% identity to the sequence as set forth in SEQ ID NO.32; or
(6) the sequence of the HCDR1 is the sequence as set forth in SEQ ID NO.35 or has at least 80%, 85%, 90%, 95% or 99% identity to the sequence as set forth in SEQ ID NO.35, the sequence of the HCDR2 is the sequence as set forth in SEQ ID NO. 17 or has at least 80%, 85%, 90%, 95% or 99% identity to the sequence as set forth in SEQ ID NO. 17, and the sequence of the HCDR3 is the sequence as set forth in SEQ ID NO.9 or has at least 80%, 85%, 90%, 95% or 99% identity to the sequence as set forth in SEQ ID NO.9; or
(7) the sequence of the HCDR1 is the sequence as set forth in SEQ ID NO.39 or has at least 80%, 85%, 90%, 95% or 99% identity to the sequence as set forth in SEQ ID NO.39, the sequence of the HCDR2 is the sequence as set forth in SEQ ID NO.40 or has at least 80%, 85%, 90%, 95% or 99% identity to the sequence as set forth in SEQ ID NO. 40, and the sequence of the HCDR3 is the sequence as set forth in SEQ ID NO.9 or has at least 80%, 85%, 90%, 95% or 99% identity to the sequence as set forth in SEQ ID NO.9; or
(8) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.40 or has at least 80%, 85%, 90%, 95% or 99% identity to the sequence as set forth in SEQ ID NO. 40, and the sequence of the HCDR3 is the sequence as set forth in SEQ ID NO.9 or has at least 80%, 85%, 90%, 95% or 99% identity to the sequence as set forth in SEQ ID NO.9; or
(9) the sequence of the HCDR1 is the sequence as set forth in SEQ ID NO.46 or has at least 80%, 85%, 90%, 95% or 99% identity to the sequence as set forth in SEQ ID NO.46, the sequence of the HCDR2 is the sequence as set forth in SEQ ID NO.23 or has at least 80%, 85%, 90%, 95% or 99% identity to the sequence as set forth in SEQ ID NO.23, and the sequence of the HCDR3 is the sequence as set forth in SEQ ID NO.9 or has at least 80%, 85%, 90%, 95% or 99% identity to the sequence as set forth in SEQ ID NO.9; or
(10) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.40 or has at least 80%, 85%, 90%, 95% or 99% identity to the sequence as set forth in SEQ ID NO.40, and the sequence of the HCDR3 is the sequence as set forth in SEQ ID NO.32 or has at least 80%, 85%, 90%, 95% or 99% identity to the sequence as set forth in SEQ ID NO.32;
wherein the sequences of the HCDRs are defined according to the IMGT definition.

In a seventh aspect, there is provided in the present application an anti-βKlotho antibody or an antigen-binding portion thereof comprising a light chain variable region comprising a light chain CDR1 (LCDR1), a light chain CDR2 (LCDR2), and a light chain CDR3 (LCDR3), wherein:
the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6;
wherein the sequences of the LCDRs are defined according to the IMGT definition.

In an eighth aspect, there is provided in the present application an isolated nucleic acid molecule encoding the anti-βKlotho antibody or antigen-binding portion thereof of the first to seventh aspects.

In a ninth aspect, there is provided in the present application a vector comprising the nucleic acid molecule of the eighth aspect.

In a tenth aspect, there is provided in the present application a host cell comprising the nucleic acid molecule of the eighth aspect or the vector of the ninth aspect.

In an eleventh aspect, there is provided in the present application an antibody-drug conjugate comprising the anti-βKlotho antibody or antigen-binding portion thereof of the first to seventh aspects conjugated with a therapeutic agent.

In a twelfth aspect, there is provided in the present application a pharmaceutical composition comprising the anti-βKlotho antibody or the antigen-binding portion thereof of the first to seventh aspects or the antibody-drug conjugate of the eleventh aspect, and a pharmaceutically acceptable carrier.

In a thirteenth aspect, there is provided in the present application the use of the anti-βKlotho antibody or antigen-binding portion thereof of the first to the seventh aspects, the nucleic acid molecule of the eighth aspect, the vector of the ninth aspect, the host cell of the tenth aspect, or the antibody-drug conjugate of the eleventh aspect in the manufacture of a medicament for the treatment of a βKlotho-mediated or βKlotho-FGFR1c-FGF21-mediated disease, for example, a metabolic disorder, such as type 1 diabetes, type 2 diabetes, dyslipidemia, non-alcoholic steatohepatitis (NASH), non-alcoholic fatty liver disease (NAFLD), cardiovascular disease, metabolic syndrome or obesity.

In a fourteenth aspect, there is provided in the present application a method of treating a βKlotho-mediated or βKlotho-FGFR1c-FGF21-mediated disease in a subject, the method comprising administering to the subject a therapeutically effective amount of the anti-βKlotho antibody or antigen-binding portion thereof of the first to seventh aspects, the antibody-drug conjugate of the eleventh aspect, or the pharmaceutical composition of the twelfth aspect. For example, the disease is a metabolic disorder, such as type 1 diabetes, type 2 diabetes, dyslipidemia, non-alcoholic steatohepatitis (NASH), non-alcoholic fatty liver disease (NAFLD), cardiovascular disease, metabolic syndrome or obesity.

In a fifteenth aspect, there is provided in the present application a conjugate comprising the anti-βKlotho antibody or antigen-binding portion thereof of the first to seventh aspects and a detectable label.

In a sixteenth aspect, there is provided in the present application a fusion protein comprising the anti-βKlotho antibody or an antigen-binding portion thereof of the first to seventh aspects.

### BRIEF DESCRIPTION OF THE DRAWINGS

FIG. 1 shows the concentration-dependeng curves of the activation of human or cynomolgus monkey FGFR1c/βKlotho receptor by antibody CB-8-42, a control antibody, and FGF19, as determined in a reporter-based assay.
FIG. 2 shows the results of body weight monitoring of cynomolgus monkeys treated with the vehicle or antibody CB-8-42.
FIG. 3 shows the results of relative body weight change monitoring in cynomolgus monkeys treated with the vehicle or antibody CB-8-42.
FIG. 4 shows the results of BMI monitoring in cynomolgus monkeys treated with the vehicle or antibody CB-8-42.
FIG. 5 shows the results of food intake monitoring of cynomolgus monkeys treated with the vehicle or antibody CB-8-42.
FIG. 6 shows changes in liver fat content in cynomolgus monkeys before and after treatment with the vehicle or antibody CB-8-42.
FIG. 7 shows changes in fasting blood glucose in cynomolgus monkeys before and after treatment with the vehicle or antibody CB-8-42.
FIG. 8 shows changes in serum insulin levels in cynomolgus monkeys before and after treatment with the vehicle or antibody CB-8-42.
FIG. 9 shows changes in serum triglyceride levels in cynomolgus monkeys before and after treatment with the vehicle or antibody CB-8-42.
FIG. 10 shows changes in serum total cholesterol levels in cynomolgus monkeys before and after treatment with the vehicle or antibody CB-8-42.
FIG. 11 shows changes in serum low density lipoprotein levels in cynomolgus monkeys before and after treatment with the vehicle or antibody CB-8-42.
FIG. 12 shows changes in serum high density lipoprotein levels in cynomolgus monkeys before and after treatment with the vehicle or antibody CB-8-42.

### DETAILED DESCRIPTION OF THE INVENTION

### DEFINITION

Unless defined otherwise, all scientific and technical terms used herein have the same meaning as understood by one of ordinary skill in the art. For definitions and terms in the art, a person skilled in the art can refer specifically to Current Protocols in Molecular Biology (Ausubel). Abbreviations for amino acid residues are standard 3-letter and/or 1-letter codes used in the art to refer to one of the 20 common L-amino acids.

Although the numerical ranges and parameter approximations are shown in broad ranges in the present application, the numerical values shown in the specific embodiments are described as accurately as possible. However, any numerical values inherently contain certain errors due to the standard deviation present in their respective measurements. Additionally, all ranges disclosed herein are to be understood as encompassing any and all subranges contained therein. For example, a range of "1 to 10" should be considered to encompass any and all subranges between the minimum value of 1 and the maximum value of 10, inclusive, i.e., all subranges starting with a minimum value of 1 or more, such as 1 to 6.1, and subranges ending with a maximum value of 10 or less, such as 5.5 to 10. Additionally, any reference referred to as "incorporated herein" is to be understood as being incorporated in its entirety.

As used herein, the term "subject" or "individual" refers to mammal, such as human, as well as other animals, such as wild animals, domestic animals, or experimental animals (e.g., gorillas, monkeys, rats, mice, rabbits, guinea pigs, woodchucks, or ground squirrels).

As used herein, the term "antigen" is a predetermined target to which an antibody can selectively bind. Examples of antigens include, but are not limited to, polypeptides, sugars, nucleic acids, lipids, haptens, or other naturally occurring or synthetic compounds.

An "antibody", in its broad sense, refer to an immunoglobulin molecule that is capable of specifically binding to a target via at least one antigen recognition site located in the variable region of the immunoglobulin molecule, and thus encompasses an intact antibody/full-length antibody, a single chain of an antibody, or any antigen binding fragment of an antibody (also referred to as an "antigen-binding portion"). When "antibody" and "antigen-binding fragment/antigen-binding portion" appear in the same context, an "antibody" can be understood as the entirety of the "antigen-binding fragment/antigen-binding portion", and they together correspond to the concept of an "antibody" in its broad sense.

As used herein, the term "βklotho" or "βklotho polypeptide" or the like include polypeptides from any vertebrate source ("polypeptide" and "protein" being interchangeable herein) or any natural βklotho. Vertebrates include mammal, such as primates (e.g. human and cynomolgus monkey (cyno)), dogs, and rodents (e.g. mice and rats), unless otherwise noted. In certain embodiments, related βklotho polypeptides are included, e.g., SNP variants, βklotho contains two domains, i.e., βklotho1 (KLB1) and βklotho 2 (KLB2). Each domain of βklotho contains a glycosyl hydrolase region. For example, the KLB1 domain of human βklotho comprises amino acid residues 1-508, and the glycosyl hydrolase region 1 comprises amino acid residues 77-508. The KLB2 domain of human βklotho comprises amino acid residues 509-1044, and the glycosyl hydrolase region 1 comprises amino acid residues 517-967. The amino acid sequence of human βklotho (NCBI Reference Sequence: NM_175737.4) is as follows:

MKPGCAAGSPGNEWIFFSTDEITTRYRNTMSNGGLQRSVILSALILLRAVTGFSGDGRAIWS
KNPNFTPVNESQLFLYDTFPKNFFWGIGTGALQVEGSWKKDGKGPSIWDHFIHTHLKNVSS
TNGSSDSYIFLEKDLSALDFIGVSFYQFSISWPRLFPDGIVTVANAKGLQYYSTLLDALVLRN
IEPIVTLYHWDLPLALQEKYGGWKNDTIIDIFNDYATYCFQMFGDRVKYWITIHNPYLVAWH
GYGTGMHAPGEKGNLAAVYTVGHNLIKAHSKVWHNYNTHFRPHQKGWLSITLGSHWIEP
NRSENTMDIFKCQQSMVSVLGWFANPIHGDGDYPEGMRKKLFSVLPIFSEAEKHEMRGTA
DFFAFSFGPNNFKPLNTMAKMGQNVSLNLREALNWIKLEYNNPRILIAENGWFTDSRVKTE
DTTAIYMMKNFLSQVLQAIRLDEIRVFGYTAWSLLDGFEWQDAYTIRRGLFYVDFNSKQKE
RKPKSSAHYYKQIIRENGFSLKESTPDVQGQFPCDFSWGVTESVLKPESVASSPQFSDPHLY
VWNATGNRLLHRVEGVRLKTRPAQCTDFVNIKKQLEMLARMKVTHYRFALDWASVLPTG
NLSAVNRQALRYYRCVVSEGLKLGISAMVTLYYPTHAHLGLPEPLLHADGWLNPSTAEAF
QAYAGLCFQELGDLVKLWITINEPNRLSDIYNRSGNDTYGAAHNLLVAHALAWRLYDRQFR
PSQRGAVSLSLHADWAEPANPYADSHWRAAERFLQFEIAWFAEPLFKTGDYPAAMREYIAS
KHRRGLSSSALPRLTEAERRLLKGTVDFCALNHFTTRFVMHEQLAGSRYDSDRDIQFLQDIT
RLSSPTRLAVIPWGVRKLLRWVRRNYGDMDIYITASGIDDQALEDDRLRKYYLGKYLQEVL
KAYLIDKVRIKGYYAFKLAEEKSKPRFGFFTSDFKAKSSIQFYNKVISSRGFPFENSSSRCSQT
QENTECTVCLFLVQKKPLIFLGCCFFSTLVLLLSIAIFQRQKRRKFWKAKNLQHIPLKKGKRV
VS (SEQ ID NO: 52)

βklotho polypeptides includes allelic variants, such as SNPs variants, splicing variants, fragments, derivatives, substitutions, deletions and insertions variants, fusion polypeptides and interspecies homologues that maintain βklotho activity and/or are sufficient to produce an anti-βklotho immune response. Those skilled in the art will recognize that the anti-βklotho antibodies provided herein may bind to βklotho polypeptides, βklotho polypeptide fragments, βklotho antigens, and/or βklotho epitopes. An epitope may be a part of a larger βklotho antigen, which may be a part of a larger βklotho polypeptide fragment, which in turn may be a part of a larger βklotho polypeptide. βklotho can exist in its natural or denatured form. The βklotho polypeptides described herein may be isolated from a variety of sources, such as human tissue types or other sources, or prepared by recombinant or synthetic methods. βklotho polypeptides may comprise a polypeptide with the same amino acid sequence as the corresponding βklotho polypeptide derived from nature. βklotho polypeptides include truncated or secreted forms(such as an extracellular domain sequence) of the βklotho polypeptide, variant forms of the polypeptide (such as a variable splicing form), and allelic variants. Orthologs of βklotho polypeptides are also well-known in the art.

The term "fibroblast growth factor" refers to a family of growth factors, including 22 members of the human FGF family. The FGF19 subfamily of fibroblast growth factors consist of human FGF21, FGF23, and FGF19, as well as mouse FGF15. The role of members of the FGF family results from their heparin-dependent binding to one or more members of the FGF receptor tyrosine kinase family. FGFRs comprise four members each having a tyrosine kinase domain, namely FGFR1, FGFR2, FGFR3, and FGFR4, and two splice variants of each of FGFR1, FGFR2, and FGFR3. These splicing variants occurring in exons 3 of FGFR1, FGFR2, and FGFR3 are named the "b" and "c" variants, such as FGFR1b, FGFR2b, FGFR3c, FGFR1c, FGFR2c, and FGFR3c, which are also referred to as FGFR1(III)b, FGFR2(III)b, FGFR3(III)b, FGFR1(III)c, FGFR2(III)c, and FGFR3(III)c, respectively.

The term "anti-βklotho antibody" or "βklotho-binding antibody" includes an antibody that is capable of binding to βklotho with sufficient affinity such that the antibody can be used as a diagnostic and/or therapeutic agent when targeting pklotho. Preferably, an anti-βklotho antibody binds to unrelated non-βklotho proteins at less than about 10% of the binding of the antibody to βklotho, as determined by, for example, fluorescence activated cell sorting (FACS) or immunoassays such as radioimmunoassays (RIA). Antibodies that "specifically bind to" or "have specificity for" βklotho are described above. In certain embodiments, the dissociation constant of an antibody described herein that binds to βklotho is less than or equal to 500 nM, 100 nM, 10 nM, 9 nM, 8 nM, 7 nM, 6 nM, 5 nM, 4 nM, 0.9 nM, 0.8 nM, 0.7 nM, 0.6 nM, 0.5 nM, 0.4 nM, 0.3 nM, 0.2 nM, or 0.1 nM. In certain embodiments, an anti-βklotho antibody binds to epitopes that are conserved in βklotho from different species, for example, between human and cynomolgus monkey βklotho.

The term "agonist antibody" is an antibody that initiates a reaction, for example, an antibody that mimics at least one functional activity of a target polypeptide, such as FGF19 or FGF21. Agonist antibodies include antibodies that are ligand mimics, for example, wherein the ligand binds to a cell surface receptor, the binding induces cell signal transduction or activity via an intracellular cell signal transduction pathway, and wherein the antibody induces similar cell signal transduction or activation.

A "full-length/intact antibody" refers to a protein comprising at least two heavy (H) chains and two light (L) chains interconnected by disulfide bonds. Each heavy chain contains a heavy chain variable region (VH) and a heavy chain constant region containing three domains CH1, CH2 and CH3. Each light chain contains a light chain variable region (VL) and a light chain constant region containing one domain CL. The VH and VL regions can be further divided into a plurality of regions with high variability, referred to as complementarity determining regions (CDRs). Among CDRs, there are more conservative regions referred to as framework regions (FRs). Each VH or VL consists of three CDRs and four FRs arranged from the amino terminus to the carboxy terminus in the following order: FR1, CDR1, FR2, CDR2, FR3, CDR3, and FR4. These variable regions of heavy and light chains contain binding domains that interact with antigens. The constant region of an antibody can mediate binding of immunoglobulins to tissues or factors in hosts, including various cells of the immune system (e.g., effector cells) and the first component of the classical complement system (Clq). A full-length/intact antibody can be any type of antibodies, such as IgD, IgE, IgG, IgA or IgM antibodies (or subclasses of the above), but not limited to any particular type. Immunoglobulins can be assigned to different classes depending on the amino acid sequences of the heavy chain constant domains of antibodies. Generally, immunoglobulins have five main classes, i.e., IgA, IgD, IgE, IgG and IgM. Several of these classes can be further classified into subclasses (isotypes), such as IgG1, IgG2, IgG3, IgG4, IgA1 and IgA2. Heavy chain constant domains corresponding to various classes of immunoglobulins are referred to as α, δ, ε, γ and µ, respectively. Subunit structures and three-dimensional structures of various classes of immunoglobulins are well known. Chimeric or humanized antibodies are also encompassed by antibodies according to the present application. It is well known to those skilled in the art that complementarity determining regions (CDRs, typically including CDR1, CDR2 and CDR3) are the subregions in variable regions that most impact on the affinity and specificity of an antibody. CDR sequences in a VH or VL can be defined in multiple common ways, including IMGT, the Chothia definition, and the Kabat definition. In embodiments of the present application, the CDR amino acid sequences are defined according to the Kabat or IMGT definition. For the variable region amino acid sequence of a given antibody, the CDR amino acid sequences in the variable region amino acid sequence can be analyzed in a variety of ways.

The term "murine antibody" refers to an antibody derived from the fusion of B cells of immunized mice with myeloma cells, the selection of murine hybridized fusion cells capable of both infinitely proliferating and secreting antibodies, and the screening, preparation and purification of the resulting antibodies. A murine antibody generally has immunogenicity and therefore needs to be subsequently humanized.

The term "humanized antibody" refers to an antibody obtained by grafting CDR sequences derived from another mammal species, such as a mouse species, onto human framework sequences. In order to retain binding affinity, some residues of the backbone segments (referred to as FR) can be modified. Humanized antibodies or fragments thereof according to the present application can be prepared by techniques known to those skilled in the art.

The term "chimeric antibody" refers to an antibody in which the variable region sequence is from one species and the constant region sequence is from another species, for example, an antibody in which the variable region sequence is from a murine antibody and the constant region sequence is from a human antibody. Chimeric antibodies or fragments thereof according to the present application can be prepared by using genetic recombination techniques. For example, a chimeric antibody can be produced by cloning a recombinant DNA comprising a promoter and a sequence encoding a variable region of a non-human, particularly murine, monoclonal antibody according to the present application, and a sequence encoding a constant region of a human antibody. Chimeric antibodies of the present application encoded by such recombinant genes will be, for example, a murine-human chimera, and its specificity is determined by the variable regions derived from murine DNA and its isoforms are determined by the constant regions derived from human DNA.

The term "partially humanized antibody" refers to an antibody containing a constant region from a human and a variable region (including CDRs) from a non-human, such as a mouse.

The term "semi-humanized antibody" is one type of humanized antibodies, and refers to an antibody in which one antibody chain comprises a murine variable region and the other antibody chain comprises a humanized variable region, i.e., a half humanized antibody.

The term "monoclonal antibody" refers to an antibody obtained from a population of substantially homogeneous antibodies. That is, individual antibodies constituting the population are the same except that naturally occurring mutations can be present in a small number of individuals.

As used herein, the term "antigen-binding fragment" or "antigen-binding portion" are used interchangeably, and in particular refer to antibody fragments such as Fv, Fab, F(ab')₂, or Fab', or any fragment which is capable of increasing the half-life by chemical modification or by incorporation into liposomes, such as addition of poly(alkylene)glycols, such as polyethylene glycol ("pegylated") (referred to as pegylated fragments Fv-PEG, scFv-PEG, Fab-PEG, F(ab')₂-PEG or Fab'-PEG) ("PEG" represents polyethylene glycol), in which the fragments have βklotho-binding activities. Preferably, a functional fragment consists of or comprises a partial sequence of the heavy or light chain variable region of the antibody from which it is derived. The partial sequence is sufficient to retain the same binding specificity and sufficient affinity as the antibody from which it is derived. Such a functional fragment can comprise at least 5 amino acids, preferably 10, 15, 25, 50 and 100 contiguous amino acids of the antibody sequence from which it is derived. Examples of antigen-binding fragments include, but are not limited to, (1) Fab fragments, which can be monovalent fragments having VL-CL chains and VH-CH1 chains; (2) F(ab')₂ fragments, which can be divalent fragments having two Fab' fragments linked by disulfide bridges of the hinge region (i.e., dimers of Fab'); and (3) Fv fragments having VL and VH domains from a single arm of an antibody.

The term "single chain fragment variable (scFv)" refers to a single polypeptide chain formed by a VH domain and a VL domain linked via a peptide linker. A (scFv)₂ comprises two VH domains linked by a peptide linker and two VL domains that are combined with the two VH domains via disulfide bridges.

The term "Fc fragment", "Fc domain", "Fc moiety" or the like refers to a portion of the constant region of an antibody heavy chain, including the hinge region, the CH2 fragment and CH3 fragment of the constant region. The Fc region of the anti-βklotho antibody may be engineered or modified, including modifications related to effector functions, such as to reduce or eliminate antibody dependent cytotoxicity and/or complement-dependent cytotoxicity, which may be achieved by introducing one or more amino acid substitutions/mutations in the Fc region of the antibody.

The term "specific binding" refers to a non-random binding reaction between two molecules, such as binding of an antibody to an antigenic epitope.

The term "multi-antibody", also referred to as "multi-specific antibody", is a molecule having binding specificity for at least two different antigens, in which a molecule that binds to only two antigens is also referred to as a dual antibody (i.e., a bispecific antibody, BsAb).

The term "bispecific antibody" refers to an antibody having binding capacities for two antigenic epitopes. The two epitopes can be on different antigens or on a same antigen. Bispecific antibodies can have a variety of structural configurations. For example, a bispecific antibody can consist of two Fc fragments and two binding moieties fused thereto, respectively (similar to a native antibody, except that the two arms bind to different antigenic targets or epitopes). An antigen binding moiety can be in the form of a single chain fragment variable (scfv) or a Fab fragment.

Generally, in order to prepare a monoclonal antibody or a functional fragment thereof, in particular a murine monoclonal antibody or a functional fragment thereof, guidelines can be found in the techniques described in "Antibodies" manual (Harlow and Lane, Antibodies: A Laboratory Manual, Cold Spring Harbor Laboratory, Cold Spring Harbor NY, pp.726, 1988) or techniques for preparing a monoclonal antibody from hybridoma cells described by Kohler and Milstein (Nature, 256: 495-497, 1975).

The term "conservative variation" or "conservative amino acid substitutions" refers to a substitution that does not substantially affect or reduce the affinity of a protein, such as the affinity of an antibody for βklotho. For example, a human antibody that specifically binds to βklotho can include up to about 1, up to about 2, up to about 5, up to about 10, or up to about 15 conservative substitutions, and specifically binds to the βklotho polypeptide. The term "conservative variation" also includes the use of a substituted amino acid in place of an unsubstituted parent amino acid as long as the antibody specifically binds to βklotho. Non-conservative substitutions are those that decrease activity or βklotho binding.

The term "isolated" biological components (e.g., nucleic acids, proteins (including antibodies) or organelles) have been substantially isolated or purified from other biological components (i.e., other chromosomal and additional chromosomal DNA and RNA, proteins and organelles) in the environment in which the components naturally occur (e.g., cells). Nucleic acids and proteins that have been "isolated" include those purified by standard purification methods. The term also includes nucleic acids and proteins prepared by recombinant expression in host cells as well as chemically synthesized nucleic acids.

The term "derived sequence" refers to a sequence that has at least 80% (preferably 85%, 90%, 95%, 98% or 99%) sequence identity to a sequence of interest and still has the same or similar function as the sequence of interest.

As used herein, the term "pharmaceutical composition" refers to a combination of at least one drug and, optionally, a pharmaceutically acceptable carrier or adjuvant that are combined together to achieve a particular purpose. In certain embodiments, the pharmaceutical composition includes a combination that is temporally and/or spatially separated, so long as it is capable of acting together to achieve the purpose of the present application. For example, the ingredients contained in the pharmaceutical composition (e.g., antibodies, nucleic acid molecules, nucleic acid molecule combinations and/or conjugates according to the present application) can be administered to a subject together or separately. When the ingredients contained in the pharmaceutical composition are separately administered to a subject, the ingredients can be administered to the subject simultaneously or sequentially. Preferably, the pharmaceutically acceptable carrier is water, a buffered aqueous solution, an isotonic salt solution such as PBS (phosphate buffer), glucose, mannitol, dextrose, lactose, starch, magnesium stearate, cellulose, magnesium carbonate, 0.3% glycerol, hyaluronic acid, ethanol, or polyalkylene glycols such as polypropylene glycol, or triglycerides. The type of a pharmaceutically acceptable carrier depends, inter alia, on whether the composition according to the present application is formulated for oral, nasal, intradermal, subcutaneous, intramuscular or intravenous administration. The compositions according to the present application can comprise wetting agents, emulsifying agents or buffer substances as additives. The pharmaceutical compositions or pharmaceutical formulations according to the present application can be administered by any suitable route, for example orally, nasally, intradermally, subcutaneously, intramuscularly or intravenously.

As used herein, "a therapeutically effective amount" or "an effective amount" refers to a dose sufficient to show benefit to a subject being administered. The actual administration dose, rate and course will depend on the condition and disease severity of the subject to be treated. The therapeutic prescription (e.g., prescribed dose) is ultimately the responsibility of and dependent on the general practitioner or other physicians, and generally takes into account the disease being treated, the condition of individual patient, the delivery site, the administration method, and other factors known to a physician.

The EC₅₀ value mainly refers to the concentration of a drug, antibody or toxin that corresponds to 50% of the maximum biological effect after a specific exposure time. Pharmaceutically, in addition to being used to characterize the ability of the activation of agonists in *in vitro* experiments, it can also be used to denote the plasma concentration required to achieve half of the maximal biological effect *in vivo.* In some literatures, EC₅₀ is also used to characterize the potency, including agonism and antagonism, of a compound at the cellular level. EC₅₀ values can be determined by methods such as ELISA.

As used herein, the term "fusion protein", in the general context, is a protein consisting of at least two domains with individual domains not associated with to each other in a natural state, and encoded by separate genes with individual genes linked to each other and transcribed and translated in an entirety, resulting in a single protein. In the technical context of the present application, a "fusion protein" comprising an antibody or antigen-binding portion refers to a product obtained by fusion of the antibody or antigen-binding portion to another biologically active protein using genetic engineering techniques, and such antibody fusion proteins have both the antigen-binding capacity of the antibody and the unique biological characteristics of the biologically active protein fused to the antibody.

The term "identity/homology/identity" in the context of an amino acid or nucleic acid sequence is defined as the percentage of identical residues in an amino acid or nucleotide sequence variant that, after alignment and introduction of gaps, achieves maximum percent homology, if desired. Methods and computer programs for alignment are well known in the art.

In a first aspect, there is provided in the present application an anti-βKlotho antibody or an antigen-binding portion thereof comprising a heavy chain variable region and a light chain variable region, wherein the heavy chain variable region comprises a heavy chain CDR1 (HCDR1), a heavy chain CDR2 (HCDR2) and a heavy chain CDR3 (HCDR3), and the light chain variable region comprises a light chain CDR1 (LCDR1), a light chain CDR2 (LCDR2) and a light chain CDR3 (LCDR3), and wherein:
(1) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.2 or has at least 80%, 85%, 90%, 95% or 99% identity to the sequence as set forth in SEQ ID NO.2, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.5 or has at least 80%, 85%, 90%, 95% or 99% identity to the sequence as set forth in SEQ ID NO.5, and the sequence of the LCDR3 is the sequence as set forth in SEQ ID NO.6 or has at least 80%, 85%, 90%, 95% or 99% identity to the sequence as set forth in SEQ ID NO.6; or
(2) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO. 14 or has at least 80%, 85%, 90%, 95% or 99% identity to the sequence as set forth in SEQ ID NO. 14, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO. 15 or has at least 80%, 85%, 90%, 95% or 99% identity to the sequence as set forth in SEQ ID NO. 15, and the sequence of the LCDR3 is the sequence as set forth in SEQ ID NO.6 or has at least 80%, 85%, 90%, 95% or 99% identity to the sequence as set forth in SEQ ID NO.6; or
(3) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.20 or has at least 80%, 85%, 90%, 95% or 99% identity to the sequence as set forth in SEQ ID NO.20, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.21 or has at least 80%, 85%, 90%, 95% or 99% identity to the sequence as set forth in SEQ ID NO.21, and the sequence of the LCDR3 is the sequence as set forth in SEQ ID NO.6 or has at least 80%, 85%, 90%, 95% or 99% identity to the sequence as set forth in SEQ ID NO.6; or
(4) the sequence of the HCDR1 is the sequence as set forth in SEQ ID NO.26 or has at least 80%, 85%, 90%, 95% or 99% identity to the sequence as set forth in SEQ ID NO.26, the sequence of the HCDR2 is the sequence as set forth in SEQ ID NO. 14 or has at least 80%, 85%, 90%, 95% or 99% identity to the sequence as set forth in SEQ ID NO. 14, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.27 or has at least 80%, 85%, 90%, 95% or 99% identity to the sequence as set forth in SEQ ID NO.27, and the sequence of the LCDR3 is the sequence as set forth in SEQ ID NO.6 or has at least 80%, 85%, 90%, 95% or 99% identity to the sequence as set forth in SEQ ID NO.6; or
(5) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.2 or has at least 80%, 85%, 90%, 95% or 99% identity to the sequence as set forth in SEQ ID NO.2, the sequence of the HCDR3 is the sequence as set forth in SEQ ID NO.31 or has at least 80%, 85%, 90%, 95% or 99% identity to the sequence as set forth in SEQ ID NO.31, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.5 or has at least 80%, 85%, 90%, 95% or 99% identity to the sequence as set forth in SEQ ID NO.5, and the sequence of the LCDR3 is the sequence as set forth in SEQ ID NO.6 or has at least 80%, 85%, 90%, 95% or 99% identity to the sequence as set forth in SEQ ID NO.6; or
(6) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO. 14 or has at least 80%, 85%, 90%, 95% or 99% identity to the sequence as set forth in SEQ ID NO. 14, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.34 or has at least 80%, 85%, 90%, 95% or 99% identity to the sequence as set forth in SEQ ID NO.34, and the sequence of the LCDR3 is the sequence as set forth in SEQ ID NO.6 or has at least 80%, 85%, 90%, 95% or 99% identity to the sequence as set forth in SEQ ID NO.6; or
(7) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.27 or has at least 80%, 85%, 90%, 95% or 99% identity to the sequence as set forth in SEQ ID NO.27, and the sequence of the LCDR3 is the sequence as set forth in SEQ ID NO.6 or has at least 80%, 85%, 90%, 95% or 99% identity to the sequence as set forth in SEQ ID NO.6; or
(8) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.42 or has at least 80%, 85%, 90%, 95% or 99% identity to the sequence as set forth in SEQ ID NO.42, and the sequence of the LCDR3 is the sequence as set forth in SEQ ID NO.6 or has at least 80%, 85%, 90%, 95% or 99% identity to the sequence as set forth in SEQ ID NO.6; or
(9) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.20 or has at least 80%, 85%, 90%, 95% or 99% identity to the sequence as set forth in SEQ ID NO.20, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.45 or has at least 80%, 85%, 90%, 95% or 99% identity to the sequence as set forth in SEQ ID NO.45, and the sequence of the LCDR3 is the sequence as set forth in SEQ ID NO.6 or has at least 80%, 85%, 90%, 95% or 99% identity to the sequence as set forth in SEQ ID NO.6; or
(10) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, the sequence of the HCDR3 is the sequence as set forth in SEQ ID NO.31 or has at least 80%, 85%, 90%, 95% or 99% identity to the sequence as set forth in SEQ ID NO.31, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.42 or has at least 80%, 85%, 90%, 95% or 99% identity to the sequence as set forth in SEQ ID NO.42, and the sequence of the LCDR3 is the sequence as set forth in SEQ ID NO.6 or has at least 80%, 85%, 90%, 95% or 99% identity to the sequence as set forth in SEQ ID NO.6;
wherein the sequences of the HCDRs and LCDRs are defined according to the Kabat definition.

In a second aspect, there is provided in the present application an anti-βKlotho antibody or an antigen-binding portion thereof comprising a heavy chain variable region and a light chain variable region, the heavy chain variable region comprises a heavy chain CDR1 (HCDR1), a heavy chain CDR2 (HCDR2) and a heavy chain CDR3 (HCDR3), and the light chain variable region comprises a light chain CDR1 (LCDR1), a light chain CDR2 (LCDR2) and a light chain CDR3 (LCDR3), wherein:
(1) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.8 or has at least 80%, 85%, 90%, 95% or 99% identity to the sequence as set forth in SEQ ID NO.8, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO. 11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(2) the sequence of the HCDR1 is the sequence as set forth in SEQ ID NO. 16 or has at least 80%, 85%, 90%, 95% or 99% identity to the sequence as set forth in SEQ ID NO. 16, the sequence of the HCDR2 is the sequence as set forth in SEQ ID NO. 17 or has at least 80%, 85%, 90%, 95% or 99% identity to the sequence as set forth in SEQ ID NO. 17, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO. 11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(3) the sequence of the HCDR1 is the sequence as set forth in SEQ ID NO.22 or has at least 80%, 85%, 90%, 95% or 99% identity to the sequence as set forth in SEQ ID NO.22, the sequence of the HCDR2 is the sequence as set forth in SEQ ID NO.23 or has at least 80%, 85%, 90%, 95% or 99% identity to the sequence as set forth in SEQ ID NO.23, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO. 11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(4) the sequence of the HCDR1 is the sequence as set forth in SEQ ID NO.28 or has at least 80%, 85%, 90%, 95% or 99% identity to the sequence as set forth in SEQ ID NO.28, the sequence of the HCDR2 is the sequence as set forth in SEQ ID NO. 17 or has at least 80%, 85%, 90%, 95% or 99% identity to the sequence as set forth in SEQ ID NO. 17, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO. 11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(5) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.8 or has at least 80%, 85%, 90%, 95% or 99% identity to the sequence as set forth in SEQ ID NO.8, the sequence of the HCDR3 is the sequence as set forth in SEQ ID NO.32 or has at least 80%, 85%, 90%, 95% or 99% identity to the sequence as set forth in SEQ ID NO. 32, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO. 11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(6) the sequence of the HCDR1 is the sequence as set forth in SEQ ID NO.3 5 or has at least 80%, 85%, 90%, 95% or 99% identity to the sequence as set forth in SEQ ID NO.35, the sequence of the HCDR2 is the sequence as set forth in SEQ ID NO. 17 or has at least 80%, 85%, 90%, 95% or 99% identity to the sequence as set forth in SEQ ID NO. 17, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO. 11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(7) the sequence of the HCDR1 is the sequence as set forth in SEQ ID NO.39 or has at least 80%, 85%, 90%, 95% or 99% identity to the sequence as set forth in SEQ ID NO.39, the sequence of the HCDR2 is the sequence as set forth in SEQ ID NO.40 or has at least 80%, 85%, 90%, 95% or 99% identity to the sequence as set forth in SEQ ID NO.40, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO. 11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(8) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.40 or has at least 80%, 85%, 90%, 95% or 99% identity to the sequence as set forth in SEQ ID NO.40, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO. 11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(9) the sequence of the HCDR1 is the sequence as set forth in SEQ ID NO.46 or has at least 80%, 85%, 90%, 95% or 99% identity to the sequence as set forth in SEQ ID NO.46, the sequence of the HCDR2 is the sequence as set forth in SEQ ID NO.23 or has at least 80%, 85%, 90%, 95% or 99% identity to the sequence as set forth in SEQ ID NO.23, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO. 11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(10) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.40 or has at least 80%, 85%, 90%, 95% or 99% identity to the sequence as set forth in SEQ ID NO.40, the sequence of the HCDR3 is the sequence as set forth in SEQ ID NO.32 or has at least 80%, 85%, 90%, 95% or 99% identity to the sequence as set forth in SEQ ID NO.32, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO. 11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6;
wherein the sequences of the HCDRs and LCDRs are defined according to the IMGT definition.

In some embodiments of the first and second aspects, the heavy chain variable region has the amino acid sequence as set forth in SEQ ID NO.12, 18, 24, 29, 33, 36, 41, 43, 47 or 49, or has an amino acid sequence having at least 80%, at least 85%, at least 90%, at least 95% or at least 99% identity to the amino acid sequence as set forth in SEQ ID NO. 12, 18, 24, 29, 33, 36, 41, 43, 47 or 49.

In some embodiments of the first and second aspects, the light chain variable region has the amino acid sequence as set forth in SEQ ID NO. 13, 19, 25, 30, 37, 44 or 48, or has an amino acid sequence having at least 80%, at least 85%, at least 90%, at least 95% or at least 99% identity to the amino acid sequence as set forth in SEQ ID NO. 13, 19, 25, 30, 37, 44 or 48.

In some embodiments of the first and second aspects,
(1) the sequence of the heavy chain variable region is the sequence as set forth in SEQ ID NO. 12, and the sequence of the light chain variable region is the sequence as set forth in SEQ ID NO. 13; or
(2) the sequence of the heavy chain variable region is the sequence as set forth in SEQ ID NO. 18, and the sequence of the light chain variable region is the sequence as set forth in SEQ ID NO. 19; or
(3) the sequence of the heavy chain variable region is the sequence as set forth in SEQ ID NO.24, and the sequence of the light chain variable region is the sequence as set forth in SEQ ID NO.25; or
(4) the sequence of the heavy chain variable region is the sequence as set forth in SEQ ID NO.29, and the sequence of the light chain variable region is the sequence as set forth in SEQ ID NO.30; or
(5) the sequence of the heavy chain variable region is the sequence as set forth in SEQ ID NO.33, and the sequence of the light chain variable region is the sequence as set forth in SEQ ID NO. 13; or
(6) the sequence of the heavy chain variable region is the sequence as set forth in SEQ ID NO.36, and the sequence of the light chain variable region is the sequence as set forth in SEQ ID NO.37; or
(7) the sequence of the heavy chain variable region is the sequence as set forth in SEQ ID NO.41, and the sequence of the light chain variable region is the sequence as set forth in SEQ ID NO.30; or
(8) the sequence of the heavy chain variable region is the sequence as set forth in SEQ ID NO.43, and the sequence of the light chain variable region is the sequence as set forth in SEQ ID NO.44; or
(9) the sequence of the heavy chain variable region is the sequence as set forth in SEQ ID NO.47, and the sequence of the light chain variable region is the sequence as set forth in SEQ ID NO.48; or
(10) the sequence of the heavy chain variable region is the sequence as set forth in SEQ ID NO.49, and the sequence of the light chain variable region is the sequence as set forth in SEQ ID NO.44.

In a third aspect, there is provided in the present application an anti-βKlotho antibody or an antigen-binding portion thereof comprising a heavy chain variable region and a light chain variable region, wherein:
the heavy chain variable region has the amino acid sequence as set forth in SEQ ID NO. 12, 18, 24, 29, 33, 36, 41, 43, 47 or 49, or has an amino acid sequence having at least 80%, at least 85%, at least 90%, at least 95% or at least 99% identity to the amino acid sequence as set forth in SEQ ID NO. 12, 18, 24, 29, 33, 36, 41, 43, 47 or 49; and/or
the light chain variable region has the amino acid sequence as set forth in SEQ ID NO. 13, 19, 25, 30, 37, 44 or 48, or has an amino acid sequence having at least 80%, at least 85%, at least 90%, at least 95% or at least 99% identity to the amino acid sequence as set forth in SEQ ID NO. 13, 19, 25, 30, 37, 44 or 48.

In a fourth aspect, there is provided in the present application an anti-βKlotho antibody or an antigen-binding portion thereof comprising a heavy chain variable region comprising a heavy chain CDR1 (HCDR1), a heavy chain CDR2 (HCDR2), and a heavy chain CDR3 (HCDR3), wherein:
(1) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.2 or has at least 80%, 85%, 90%, 95% or 99% identity to the sequence as set forth in SEQ ID NO.2, and the sequence of the HCDR3 is the sequence as set forth in SEQ ID NO.3 or has at least 80%, 85%, 90%, 95% or 99% identity to the sequence as set forth in SEQ ID NO.3; or
(2) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.14 or has at least 80%, 85%, 90%, 95% or 99% identity to the sequence as set forth in SEQ ID NO.14, and the sequence of the HCDR3 is the sequence as set forth in SEQ ID NO.3 or has at least 80%, 85%, 90%, 95% or 99% identity to the sequence as set forth in SEQ ID NO.3; or
(3) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.20 or has at least 80%, 85%, 90%, 95% or 99% identity to the sequence as set forth in SEQ ID NO. 20, and the sequence of the HCDR3 is the sequence as set forth in SEQ ID NO.3 or has at least 80%, 85%, 90%, 95% or 99% identity to the sequence as set forth in SEQ ID NO.3; or
(4) the sequence of the HCDR1 is the sequence as set forth in SEQ ID NO.26 or has at least 80%, 85%, 90%, 95% or 99% identity to the sequence as set forth in SEQ ID NO.26, the sequence of the HCDR2 is the sequence as set forth in SEQ ID NO.14 or has at least 80%, 85%, 90%, 95% or 99% identity to the sequence as set forth in SEQ ID NO.14, and the sequence of the HCDR3 is the sequence as set forth in SEQ ID NO.3 or has at least 80%, 85%, 90%, 95% or 99% identity to the sequence as set forth in SEQ ID NO.3; or
(5) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.2 or has at least 80%, 85%, 90%, 95% or 99% identity to the sequence as set forth in SEQ ID NO.2, and the sequence of the HCDR3 is the sequence as set forth in SEQ ID NO.31 or has at least 80%, 85%, 90%, 95% or 99% identity to the sequence as set forth in SEQ ID NO.31; or
(6) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, and the sequence of the HCDR3 is the sequence as set forth in SEQ ID NO.3 or has at least 80%, 85%, 90%, 95% or 99% identity to the sequence as set forth in SEQ ID NO.3; or
(7) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, and the sequence of the HCDR3 is the sequence as set forth in SEQ ID NO.31 or has at least 80%, 85%, 90%, 95% or 99% identity to the sequence as set forth in SEQ ID NO.31;
wherein the sequences of the HCDRs are defined according to the Kabat definition.

In a fifth aspect, there is provided in the present application an anti-βKlotho antibody or an antigen-binding portion thereof comprising a light chain variable region comprising a light chain CDR1 (LCDR1), a light chain CDR2 (LCDR2), and a light chain CDR3 (LCDR3), wherein:
(1) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.5 or has at least 80%, 85%, 90%, 95% or 99% identity to the sequence as set forth in SEQ ID NO.5, and the sequence of the LCDR3 is the sequence as set forth in SEQ ID NO.6 or has at least 80%, 85%, 90%, 95% or 99% identity to the sequence as set forth in SEQ ID NO.6; or
(2) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO. 15 or has at least 80%, 85%, 90%, 95% or 99% identity to the sequence as set forth in SEQ ID NO. 15, and the sequence of the LCDR3 is the sequence as set forth in SEQ ID NO.6 or has at least 80%, 85%, 90%, 95% or 99% identity to the sequence as set forth in SEQ ID NO.6; or
(3) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.21 or has at least 80%, 85%, 90%, 95% or 99% identity to the sequence as set forth in SEQ ID NO.21, and the sequence of the LCDR3 is the sequence as set forth in SEQ ID NO.6 or has at least 80%, 85%, 90%, 95% or 99% identity to the sequence as set forth in SEQ ID NO.6; or
(4) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.27 or has at least 80%, 85%, 90%, 95% or 99% identity to the sequence as set forth in SEQ ID NO.27, and the sequence of the LCDR3 is the sequence as set forth in SEQ ID NO.6 or has at least 80%, 85%, 90%, 95% or 99% identity to the sequence as set forth in SEQ ID NO.6; or
(5) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.34 or has at least 80%, 85%, 90%, 95% or 99% identity to the sequence as set forth in SEQ ID NO.34, and the sequence of the LCDR3 is the sequence as set forth in SEQ ID NO.6 or has at least 80%, 85%, 90%, 95% or 99% identity to the sequence as set forth in SEQ ID NO.6; or
(6) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.42 or has at least 80%, 85%, 90%, 95% or 99% identity to the sequence as set forth in SEQ ID NO.42, and the sequence of the LCDR3 is the sequence as set forth in SEQ ID NO.6 or has at least 80%, 85%, 90%, 95% or 99% identity to the sequence as set forth in SEQ ID NO.6; or
(7) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.45 or has at least 80%, 85%, 90%, 95% or 99% identity to the sequence as set forth in SEQ ID NO.45, and the sequence of the LCDR3 is the sequence as set forth in SEQ ID NO.6 or has at least 80%, 85%, 90%, 95% or 99% identity to the sequence as set forth in SEQ ID NO.6;
wherein the sequences of the LCDRs are defined according to the Kabat definition.

In a sixth aspect, there is provided in the present application an anti-βKlotho antibody or an antigen-binding portion thereof comprising a heavy chain variable region comprising a heavy chain CDR1 (HCDR1), a heavy chain CDR2 (HCDR2) and a heavy chain CDR3 (HCDR3), wherein:
(1) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth inSEQ ID NO.8 or has at least 80%, 85%, 90%, 95% or 99% identity to the sequence as set forth in SEQ ID NO.8, and the sequence of the HCDR3 is the sequence as set forth in SEQ ID NO.9 or has at least 80%, 85%, 90%, 95% or 99% identity to the sequence as set forth in SEQ ID NO.9; or
(2) the sequence of the HCDR1 is the sequence as set forth in SEQ ID NO. 16 or has at least 80%, 85%, 90%, 95% or 99% identity to the sequence as set forth in SEQ ID NO. 16, the sequence of the HCDR2 is the sequence as set forth in SEQ ID NO. 17 or has at least 80%, 85%, 90%, 95% or 99% identity to the sequence as set forth in SEQ ID NO. 17, and the sequence of the HCDR3 is the sequence as set forth in SEQ ID NO.9 or has at least 80%, 85%, 90%, 95% or 99% identity to the sequence as set forth in SEQ ID NO.9; or
(3) the sequence of the HCDR1 is the sequence as set forth in SEQ ID NO.22 or has at least 80%, 85%, 90%, 95% or 99% identity to the sequence as set forth in SEQ ID NO.22, the sequence of the HCDR2 is the sequence as set forth in SEQ ID NO.23 or has at least 80%, 85%, 90%, 95% or 99% identity to the sequence as set forth in SEQ ID NO. 23, and the sequence of the HCDR3 is the sequence as set forth in SEQ ID NO.9 or has at least 80%, 85%, 90%, 95% or 99% identity to the sequence as set forth in SEQ ID NO.9; or
(4) the sequence of the HCDR1 is the sequence as set forth in SEQ ID NO.28 or has at least 80%, 85%, 90%, 95% or 99% identity to the sequence as set forth in SEQ ID NO.28, the sequence of the HCDR2 is the sequence as set forth in SEQ ID NO. 17 or has at least 80%, 85%, 90%, 95% or 99% identity to the sequence as set forth in SEQ ID NO. 17, and the sequence of the HCDR3 is the sequence as set forth in SEQ ID NO.9 or has at least 80%, 85%, 90%, 95% or 99% identity to the sequence as set forth in SEQ ID NO.9; or
(5) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.8 or has at least 80%, 85%, 90%, 95% or 99% identity to the sequence as set forth in SEQ ID NO.8, and the sequence of the HCDR3 is the sequence as set forth in SEQ ID NO.32 or has at least 80%, 85%, 90%, 95% or 99% identity to the sequence as set forth in SEQ ID NO.32; or
(6) the sequence of the HCDR1 is the sequence as set forth in SEQ ID NO.35 or has at least 80%, 85%, 90%, 95% or 99% identity to the sequence as set forth in SEQ ID NO.35, the sequence of the HCDR2 is the sequence as set forth in SEQ ID NO. 17 or has at least 80%, 85%, 90%, 95% or 99% identity to the sequence as set forth in SEQ ID NO. 17, and the sequence of the HCDR3 is the sequence as set forth in SEQ ID NO.9 or has at least 80%, 85%, 90%, 95% or 99% identity to the sequence as set forth in SEQ ID NO.9; or
(7) the sequence of the HCDR1 is the sequence as set forth in SEQ ID NO.39 or has at least 80%, 85%, 90%, 95% or 99% identity to the sequence as set forth in SEQ ID NO.39, the sequence of the HCDR2 is the sequence as set forth in SEQ ID NO.40 or has at least 80%, 85%, 90%, 95% or 99% identity to the sequence as set forth in SEQ ID NO. 40, and the sequence of the HCDR3 is the sequence as set forth in SEQ ID NO.9 or has at least 80%, 85%, 90%, 95% or 99% identity to the sequence as set forth in SEQ ID NO.9; or
(8) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.40 or has at least 80%, 85%, 90%, 95% or 99% identity to the sequence as set forth in SEQ ID NO. 40, and the sequence of the HCDR3 is the sequence as set forth in SEQ ID NO.9 or has at least 80%, 85%, 90%, 95% or 99% identity to the sequence as set forth in SEQ ID NO.9; or
(9) the sequence of the HCDR1 is the sequence as set forth in SEQ ID NO.46 or has at least 80%, 85%, 90%, 95% or 99% identity to the sequence as set forth in SEQ ID NO.46, the sequence of the HCDR2 is the sequence as set forth in SEQ ID NO.23 or has at least 80%, 85%, 90%, 95% or 99% identity to the sequence as set forth in SEQ ID NO. 23, and the sequence of the HCDR3 is the sequence as set forth in SEQ ID NO.9 or has at least 80%, 85%, 90%, 95% or 99% identity to the sequence as set forth in SEQ ID NO.9; or
(10) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.40 or has at least 80%, 85%, 90%, 95% or 99% identity to the sequence as set forth in SEQ ID NO. 40, and the sequence of the HCDR3 is the sequence as set forth in SEQ ID NO.32 or has at least 80%, 85%, 90%, 95% or 99% identity to the sequence as set forth in SEQ ID NO.32;
wherein the sequences of the HCDRs are defined according to the IMGT definition.

In a seventh aspect, there is provided in the present application an anti-βKlotho antibody or an antigen-binding portion thereof comprising a light chain variable region comprising a light chain CDR1 (LCDR1), a light chain CDR2 (LCDR2), and a light chain CDR3 (LCDR3), wherein:
the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6;
wherein the sequences of the LCDRs are defined according to the IMGT definition.

In some embodiments of the first to seventh aspects, an anti-βKlotho antibody or an antigen-binding portion thereof binds to human βKlotho. In some embodiments, the anti-βKlotho antibody or antigen-binding portion thereof specifically binds to human βKlotho.

In some embodiments of the first to seventh aspects, the anti-βKlotho antibody is an intact antibody, a single chain fragment variable (scFv), a bispecific antibody, or a multispecific antibody

In some embodiments of the first to seventh aspects, the antigen-binding portion of the anti-βKlotho antibody is a Fab, Fab', Fv or F(ab')₂.

In some embodiments of the first to seventh aspects, the anti-βKlotho antibody is a murine antibody, a chimeric antibody, a humanized antibody, or a fully human antibody.

In some embodiments of the first to seventh aspects, the anti-βKlotho antibody is a monoclonal antibody.

In some embodiments of the first to seventh aspects, the anti-βKlotho antibody is of IgM, IgD, IgA, IgA, or IgE type. In some embodiments, the anti-βKlotho antibody is an IgG antibody. In some more particular embodiments, the anti-βKlotho antibody is an IgG1 antibody.

In some embodiments of the first to seventh aspects, the anti-βKlotho antibody is of IgG1, IgG2, or IgG4 isotype.

In some embodiments of the first to seventh aspects, the anti-βKlotho antibody is of IgG1/IgG4, IgG2/IgG4 or IgG1/IgG2 chimeric type.

In some embodiments of the first to seventh aspects, the anti-βKlotho antibody comprises a kappa-isoform or lambda-isoform light chain constant region.

In some embodiments of the first to seventh aspects, the anti-βKlotho antibody comprises a human IgG1 heavy chain constant region and a human kappa light chain constant region.

In some embodiments of the first to seventh aspects, an anti-βKlotho antibody or an antigen-binding portion thereof is for use in medical therapy.

In some embodiments of the first to seventh aspects, the anti-βKlotho antibody or antigen-binding portion thereof has ADCC or CDC activity.

In some embodiments of the first to seventh aspects, the anti-βKlotho antibody comprises a wild-type Fc region.

In some embodiments of the first to seventh aspects, the anti-βKlotho antibody or antigen-binding portion thereof is engineered to have enhanced ADCC or CDC activity.

In some embodiments of the first to seventh aspects, the anti-βKlotho antibody or antigen-binding portion thereof is engineered to have attenuated ADCC or CDC activity.

For the target βKlotho and the *in vivo* application of the antibody considered by the present application, an antibody with attenuated or even eliminated ADCC or CDC activity may be advantageous because an excessively strong FC effect may affect or even weaken the efficacy of the antibody. The methods of attenuating or eliminating the Fc effect are known in the art, for example, 1) amino acid residue mutation modification (mainly attenuating binding to related receptors), 2) glycosylation modification, and 3) IgG4 antibody modification.

In some embodiments of the first to seventh aspects, the anti-βKlotho antibody has a heavy chain variable region as set forth in SEQ ID NO.49, a light chain variable region as set forth in SEQ ID NO.44, a heavy chain constant region as set forth in SEQ ID NO.50, and the light chain constant region as set forth in SEQ ID NO.51.

In an eighth aspect, there is provided in the present application an isolated nucleic acid molecule encoding the anti-βKlotho antibody or antigen-binding portion thereof of the first to seventh aspects. In some embodiments, there is provided in the present application a combination of isolated polynucleotides comprising a polynucleotide encoding a light chain of an antibody of the present application or an antigen-binding portion thereof and a polynucleotide encoding the heavy chain of the antibody of the present application or antigen-binding portion. In some embodiments, the polynucleotide is operably linked to a regulatory sequence that can be recognized by host cells transformed with a vector.

In a ninth aspect, there is provided in the present application a vector (e.g., an expression vector) comprising the nucleic acid molecule or the combination of polynucleotides of the eighth aspect.

In some embodiments, an expression vector of the present application comprises a nucleic acid molecule or a combination of polynucleotides as described herein operably linked to a regulatory sequence that allows expression of the polypeptide encoded by the polynucleotides in a host cell or a cell-free expression system. The selection of an expression vector depends on the selection of host cells, and can be selected so as to have the desired expression and regulatory characteristics in the selected host cells.

An "expression vector" is a vector comprising one or more expression regulatory sequences. An "expression regulatory sequence" is a DNA sequence that controls and regulates transcription and/or translation of another DNA sequence.

The nucleic acid in the vector can be operably linked to one or more expression regulatory sequences. As used herein, the term "operably linked" means incorporation into a genetic construct such that the expression regulatory sequence effectively controls the expression of the target coding sequence. Examples of expression regulatory sequences include promoters, enhancers, and transcription termination regions. A promoter is an expression regulatory sequence consisting of a region of a DNA molecule that is typically within 100 nucleotides upstream of the transcriptional initiation site (typically in the vicinity of the start site of RNA polymerase II). In order for the coding sequence to be under the control of the promoter, the translation initiation site of the polypeptide translation reading frame must be located between 1 and about 50 nucleotides downstream of the promoter. Enhancers provide expression specificity in terms of time, position and level. Unlike promoters, enhancers can function at different distances from the transcription site. Enhancers can also be located downstream of the transcription initiation site. When an RNA polymerase is capable of transcribing a coding sequence into an mRNA, which can then be translated into a protein encoded by the coding sequence, the coding sequence is "operably linked" to an expression regulatory sequence in a cell and is under "control" of the expression regulatory sequence.

Suitable expression vectors include, but are not limited to, plasmids and viral vectors derived from, for example, phages, baculoviruses, tobacco mosaic viruses, herpes viruses, cytomegaloviruses, retroviruses, vaccinia viruses, adenoviruses, and adeno-associated viruses. Many vectors and expression systems are commercially available from companies such as Novagen (Madison, WI), Clontech (Palo Alto, CA), Stratagene (LaJolla, CA), and Invitrogen Life Technologies (Carlsbad, CA).

An expression vector can comprise a tag sequence. A tag sequence is generally expressed as a fusion with the encoded polypeptide. Such tags can be inserted at any position within the polypeptide, including the carboxyl or amino terminus. Examples of useful tags include, but are not limited to, Fc fragments, polyhistidine, green fluorescent protein (GFP), glutathione S-transferase (GST), c-myc, hemagglutinin, FlagTM tags (Kodak, NewHaven, CT), maltose E-binding protein, and protein A. In some embodiments, the nucleic acid molecule encoding the βKlotho fusion polypeptide is present in a vector comprising a nucleic acid encoding one or more domains of an Ig heavy chain constant region, e.g., an amino acid sequence (Fc fragment) corresponding to the hinge region, CH2 region, and CH3 region of the human immunoglobulin Cγ1 chain.

In a tenth aspect, there is provided in the present application a host cell comprising the nucleic acid molecule of the eighth aspect or the vector of the ninth aspect. In some embodiments, the host cell can be a prokaryotic host cell, a eukaryotic host cell, or a phage. The prokaryotic host cell can be *Escherichia coli, Bacillus subtilis, Streptomyces or Proteus mirabilis.* The eukaryotic host cell can be a fungus such as *Pichia pastoris, Saccharomyces cerevisiae, Schizosaccharomyces cerevisiae,* or *Trichoderma,* an insect cell such as grass armyworm, or a plant cell such as tobacco, or a mammal cell such as a BHK cell, a CHO cell, a COS cell, or a myeloma cell. In some embodiments, the host cells described herein are preferably mammal cells, more preferably BHK cells, CHO cells, NSO cells, or COS cells.

In an eleventh aspect, there is provided in the present application an antibody-drug conjugate, also referred to as an ADC, comprising the anti-βKlotho antibody or antigen-binding portion thereof according to any one embodiment of the first to seventh aspects conjugated with a therapeutic agent.

In a twelfth aspect, there is provided in the present application a pharmaceutical composition comprising an anti-βKlotho antibody or an antigen-binding portion thereof of the first to seventh aspects or an antibody-drug conjugate of the eleventh aspect, and a pharmaceutically acceptable carrier.

In some embodiments, the pharmaceutical composition is for the treatment of a βKlotho-mediated or βKlotho-FGFR1c-FGF21-mediated disease, such as a disease that requires a treatment that simulates or enhances the *in vivo* effects of FGF19 and/or FGF21. In some specific embodiments, the disease a metabolic disorder, such as type 1 diabetes, type 2 diabetes, dyslipidemia, non-alcoholic steatohepatitis (NASH), non-alcoholic fatty liver disease (NAFLD), cardiovascular disease, metabolic syndrome or obesity.

In some embodiments, the pharmaceutical composition can further comprise one or more of lubricants (such as talc, magnesium stearate, and mineral oil), wetting agents, emulsifiers, suspending agents, preservatives (such as benzoic acid, sorbic acid and calcium propionate), sweetening agents and/or flavoring agents. In some embodiments, the pharmaceutical compositions herein can be formulated as tablets, pills, powders, lozenges, elixirs, suspensions, emulsions, solutions, syrups, suppositories, or capsules.

In some embodiments, the pharmaceutical compositions of the present application can be delivered using any physiologically acceptable mode of administration including, but not limited to, oral administration, parenteral administration, nasal administration, rectal administration, intraperitoneal administration, intravascular injection, subcutaneous administration, transdermal administration, and inhalation administration.

In some embodiments, the pharmaceutical composition for therapeutic use can be formulated for storage in a lyophilized formulation or in the form of an aqueous solution by mixing the agents having the desired purity with pharmaceutically acceptable carriers or excipients, where appropriate.

In a thirteenth aspect, there is provided in the present application the use of the anti-βKlotho antibody or antigen-binding portion thereof of the first to seventh aspects, the nucleic acid molecule of the eighth aspect, the vector of the ninth aspect, the host cell of the tenth aspect, or the antibody-drug conjugate of the eleventh aspect for the manufacture of a medicament for the treatment of a βKlotho-mediated or βKlotho-FGFR1c-FGF21-mediated disease such as a disease that requires a treatment that simulates or enhances the *in vivo* effects of FGF19 and/or FGF21. In some specific embodiments, the disease is a metabolic disorder, such as type 1 diabetes, type 2 diabetes, dyslipidemia, non-alcoholic steatohepatitis (NASH), non-alcoholic fatty liver disease (NAFLD), cardiovascular disease, metabolic syndrome or obesity.

In a fourteenth aspect, there is provided in the present application a method of treating a βKlotho-mediated or βKlotho-FGFR1c-FGF21-mediated disease (such as a disease that requires a treatment that simulates or enhances the *in vivo* effects of FGF19 and/or FGF21) in a subject, comprising administering to the subject a therapeutically effective amount of an anti-βKlotho antibody or antigen-binding portion thereof of the first to seventh aspects, the antibody-drug conjugate of the eleventh aspect, or the pharmaceutical composition of the twelfth aspect. In some particular embodiments, the disease is a metabolic disorder including, but not limited to, type 1 diabetes, type 2 diabetes, dyslipidemia, non-alcoholic steatohepatitis (NASH), non-alcoholic fatty liver disease (NAFLD), cardiovascular disease, metabolic syndrome, obesity, or any disease, disorder, or condition that generally requires a treatment that simulates or enhances the *in vivo* effects of FGF19 and/or FGF21. In some embodiments of a method of treating or preventing a disease, disorder, or condition, the method comprises exposing cells *in vitro* to an anti-βklotho antibody.

In some embodiments of the twelfth to fourteenth aspects, the anti-βKlotho antibody or antigen-binding portion thereof of the present application may be used alone or in combination with other compositions in therapy. For example, the anti-βklotho antibody may be co-administered with at least one additional therapeutic agent and/or adjuvant. In some embodiments, additional compounds are therapeutic antibodies other than anti-βklotho antibodies, including, but not limited to, drugs for the treatment of diabetes mellitus, dyslipidemia, NASH, NAFLD, cardiovascular disease, metabolic syndrome or obesity, such as biguanides, sulfonylureas, thiazolidinediones, GLP-1 analogues, PPARgamma agonists, dipeptidyl peptidase-4 (DPP-4) inhibitors, bromocriptine formulations, bile acid chelating agents, insulin, alpha glucosidase inhibitors, SGLT-2 inhibitors, appetite-suppressing, and weight-reducing drugs, anti-inflammatory drugs such as aspirin and ibuprofen, and so on.

In a fifteenth aspect, there is provided in the present application a conjugate comprising the anti-βKlotho antibody or antigen-binding portion thereof of the first to seventh aspects and a detectable label. The detectable label is, for example, a detectable fluorescent label, a chemiluminescent label, or an isotopic label.

In a sixteenth aspect, there is provided in the present application a fusion protein comprising the anti-βKlotho antibody or antigen-binding portion thereof of the first to seventh aspects. Examples of antibody fusion proteins include Fab fusion proteins, Fc fusion proteins and single chain fragment variable (scFv) fusion proteins, which are named depending on the site to which the effector protein (e.g., cytokine) is fused.

In some embodiments of the fifteenth or sixteenth aspect, the conjugate may be formed by synthesizing an anti-βklotho antibody of the present application that covalently binds to one or more non-antibody acting agents via a linker. In some embodiments, the antibody of the present application is conjugated or recombinantly fused with a diagnostic, detection, or therapeutic agent or any other molecule. Conjugated or recombinantly fused antibodies may be used, for example, to monitor or predict the onset, occurrence, progression, and/or severity of a βklotho-mediated disease as part of a clinical trial approach, such as to determine the efficacy of a particular therapy. Such diagnosis and detection may be accomplished, for example, by conjugating an antibody with a detectable marker. Detectable markers include, but are not limited to, enzymes, prost hetic groups, fluorescent markers, chemiluminescent markers and isotope markers. In some embodiments, the antibody of the present application may be conjugated or fused with a therapeutic portion or a drug portion (which may also be the antibody-drug conjugate of the eleventh aspect) that modifies a specified biological response. The therapeutic or pharmaceutical portion is not limited to typical chemotherapeutic agents. For example, the drug moiety may be a protein, peptide, polypeptide, or small molecule toxin having the desired biological activity.

It is to be understood that the foregoing detailed description is intended only to enable those skilled in the art to have a better understanding of the present application and is not intended to be limiting in any way. Various modifications and alterations can be made to the described embodiments by those skilled in the art.

### EXAMPLES

Specific embodiments of the present application will be described in detail below in connection with the Examples, but those skilled in the art will appreciate that the following Examples are merely illustrative of the inventions of the present application and should not be construed as limiting the scope of the present application.

### EXAMPLE 1: Antibody Discovery Based on Phage Display

In order to obtain antibodies that specifically bind to human βKlotho, the inventors performed phage display work based on a human natural single chain antibody phage library.

Firstly, human βKlotho extracellular segment protein (R&D systems, 5889-KB-050) was biotinylated for liquid phase panning. Then, ProX specific antibodies were screened using human natural single chain antibody phage display library (Shanghai Ruizhi Chemical Research Co., Ltd.). For each round of panning, the library was first incubated with Dynabeads streptavidin beads to remove non-specific conjugates of Dynabeads beads. The library was then panned with the biotinylated human βKlotho extracellular segment proteins to enrich human βKlotho-specific phage antibodies. Non-specific phage antibodies were removed by multiple washings, and phage antibodies that specifically bind to human βKlotho were recovered by elution with trypsin or a known βKlotho antibody (Tab hIgG1 antibody, 5H23 from NGM Patent CN 201580016315.8). The eluted phage infects *Escherichia coli* TG1 with the aid of helper phage M13KO7 (New England BioLabs.Inc.) to generate a library for the next round of panning. Typically, each round of panning includes negative selection, positive selection, washing, elution, and amplification. After each round of panning, if most of the output clones are non-duplicate single clones, panning will continue for up to 3 rounds based on sequencing results. Otherwise, if the proportion of repeated sequences is high, the panning ends in this round. Phage output from each round will be retained for subsequent use.

Monoclones were selected from the panned library and grown overnight in 2YT medium containing 100 µg/mL ampicillin and 2% glucose. Overnight cultured monoclones were transferred to 2YT medium containing 100 µg/mL ampicillin. Induced expression of an scFv was performed at a final IPTG concentration of 1 mM and at 30°C for 16 hours. The supernatant was taken from the centrifuged bacterial liquid and positive clones were screened using the following method.

ELISA-based Binding Assay: 100 µL of 1µg/mL streptavidin was added to a 96-well plate, and the plate was coated overnight at 4°C. After blocking at room temperature, 50 µL of 0.5 µg/mL biotinylated human βKlotho extracellular segment protein was added for incubation at room temperature. After washing, 60 µL of blocking solution was added for incubation at room temperature. After incubation with 40 µL of scFv supernatant at room temperature, the control wells were supplemented with Tab hIgG1 antibody and hIgG1 isotype control at a final concentration of 1 µg/mL, respectively. After washing, anti-Myc-HRP and anti-hIgG-Fc-specific-HRP were added and incubated at room temperature. After washing, 100 µL of 3,3',5,5'-tetramethylbenzidine (TMB) substrate solution was added and the plate was incubated at room temperature in dark for 15 minutes. 100 µL of 1 M HCl was added to stop the reaction, and the absorbance was read at 450 nm.

ELISA-based Competition Assay: 100 µL of 1 µg/mL streptavidin was added to a 96-well plate, and the plate was coated overnight at 4°C. After blocking at room temperature, 50 µL of 0.5 µg/mL biotinylated human βKlotho extracellular protein was added for incubation at room temperature. After washing, 60 µL of 3 µg/mL Tab hIgG1 was added for incubation at room temperature (a blocking solution was added to the Tab hIgG1 and hIgG1 isotype antibody control wells). Then, 40 µL of scFv supernatant was added for incubation at room temperature, and Tab hIgG1 and hIgG1 isotype antibodies at a final concentration of 1 µg/mL were added to the control wells. After washing, anti-Myc-HRP and anti-hIgG-Fc-specific-HRP were added for incubation at room temperature. After washing, 100 µL of TMB was added for incubation at room temperature in dark for 15 minutes. 100 µL of 1 M HCl was added to stop the reaction, and the absorbance was read at 450 nm.

According to result analysis, if an antibody clone satisfies the condition OD₄₅₀ >0.4 in the binding ELISA and OD 450 (binding ELISA)/OD 450 (competition ELISA) ratio ≥2, the antibody clone is considered capable of specifically binding to human βKlotho and its antigen-binding epitope is similar or identical to that of Tab hIgG1 antibody. If an antibody clone satisfies the condition OD₄₅₀ >0.4 in the binding ELISA and OD₄₅₀ (binding ELISA)/OD 450 (competition ELISA) ratio <2, the antibody clone is considered binding to an epitope different from that of Tab hIgG1 antibody. The antibody clones having binding epitopes similar or identical to that of Tab hIgG1 antibody and having binding epitopes partially different from that of Tab hIgG1 antibody were selected for sequencing. Finally, twenty-two variable region sequences of antibodies binding to human βKlotho were obtained, named CB-1, CB-2, CB-4, CB-5, CB-6, CB-8, CB-9, CB-10, CB-11, CB-14, CB-16, CB-17, CB-18, CB-19, CB-21, CB-22, CB-23, CB-24, CB-25, CB-26, CB-27, and CB-28, respectively. Then, full-length antibodies comprising the above antibody variable regions were prepared using the human heavy chain IgG1 constant region and the human light chain lambda or kappa constant region.

### EXAMPLE 2: Screening of antibodies capable of activating human or monkey FGFR1c/βKlotho receptors using a reporter-based assay

The abilities of the full-length antibodies described in Example 1 to activate the human FGFR1c/βKlotho receptor were determined based on a reporter-based assay. This method is applied to stable transgenic cell line. After the human FGFR1c/βKlotho receptor is activated by FGF19 or an antibody, it facilitates the downstream signaling pathway, leading to ERK phosphorylation. The ERK Phosphorylation further leads to the phosphorylation of Gal4-Elk1 and binding to the USA promoter region on a DNA. The transcription of a luciferase reporter gene is then primed, allowing for the determination of the activation of FGF19 or an antibody on the FGFR1c/βKlotho receptor by measuring luciferase levels.

The following method was used to construct the stable transgenic cell line with a reporter gene. Firstly, two reporter-carrying plasmids Gal4-Elk1 and 5xUAS-Luc were transferred into rat L6 cells, and cell lines stably expressing the target molecules were obtained after antibiotic selection. Then, plasmids encoding human FGFR1c and βKlotho genes or cynomolgus monkey FGFR1c and βKlotho genes were further transfected into the cells, and FGFR1c/βKlotho/Gal4-Elk1/Luc stable transgenic cell lines were obtained after several rounds of antibiotic pressure selection.

Activation of the human FGFR1c/βKlotho receptor by an antibody was determined using the following method. 40µl of an antibody or FGF19 solution with its concentration three times the final concentration was added to a 96- well plate. Then, an 80 µl volume of the stable transgenic cell at an appropriate density was added or mixed with the antibody. The mixture was then incubated overnight in an incubator at 37°C with 5% CO₂ and saturated humidity. Luciferase levels in the cells were determined the following day using the One-Glo (Promega) kit.

The data measured for activation of human or monkey FGFR1c/βKlotho receptor by the antibodies are shown in the following table.

**Table 1: Data for Activation of Human or Monkey FGFR1c/βKlotho Receptor by Antibodies**

| **Sample** | **Human βKlotho/FGFR1c** | **Cynomolgus monkey βKlotho/FGFR1c** |
|---|---|---|
| | **EC₅₀(nM)** | **EC₅₀(nM)** |
| CB-1 | 19.7 | >100 |
| CB-6 | 4.80 | >100 |
| CB-8 | 1.31 | 4.89 |
| CB-10 | 11.3 | >100 |
| CB-14 | >100 | Not determined |
| CB-16 | 30.5 | Not determined |
| CB-17 | >100 | Not determined |
| CB-18 | >100 | Not determined |
| CB-19 | >100 | Not determined |
| CB-21 | >100 | Not determined |
| CB-22 | >100 | Not determined |
| CB-23 | 7.27 | 35 |
| CB-24 | >100 | Not determined |
| CB-25 | >100 | Not determined |
| CB-26 | >100 | Not determined |
| CB-27 | >100 | Not determined |
| CB-28 | 28.8 | 107 |

| | | |
|---|---|---|
| Note: "Not determined" means determination was not carried out. | | |

As shown in the results in Table 1, some anti-human βKlotho antibodies obtained by the phage display technology screening in the present application had activation effects on human and/or cynomolgus monkey βKlotho/FGFR1c.

### EXAMPLE 3: Detection of Binding of Antibodies to Human βKlotho, Human αKlotho and Mouse βKlotho by ELISA

In this Example, the binding of some antibodies in Example 1 to human βKlotho, human αKlotho, and mouse βKlotho was detected using an ELISA assay as described below. A solution of human βKlotho (R&D systems), human αKlotho (R&D systems), or mouse βKlotho (R&D systems) extracellular segment protein at a specified concentration was added to a nickel-coated 96-well plate (Pierce) and the plate was incubated overnight at room temperature. The next day, the plate was blocked with PBS/1% BSA, and then antibodies at various concentrations were added to the wells. After incubating the plate for 1.5 hours at room temperature, a HRP-labeled anti-human IgG Fab secondary antibody was added to the wells and the plate was incubated for 30 minutes at room temperature. After the substrate was added and reacted at room temperature for 15 minutes, the reaction-stopping solution was added. OD₄₅₀ values were read. Exemplary data of antibodies' binding to human βKlotho, human αKlotho, and mouse βKlotho in the ELISA assay are as follows.

**Table 2: Data for Binding of Antibodies to Human βKlotho, Human αKlotho, and Mouse βKlotho**

| Sample | Human βKlotho | Human βKlotho | Mouse βKlotho |
|---|---|---|---|
| | EC₅₀ (nM) | EC₅₀ (nM) | EC₅₀ (nM) |
| CB-1 | 1.10 | >100 | >100 |
| CB-6 | 0.22 | >100 | >100 |
| CB-8 | 1.60 | >100 | >100 |
| CB-10 | 0.26 | 40 | 57 |
| CB-16 | 1.85 | Not determined | Not determined |
| CB-23 | 0.65 | Not determined | Not determined |
| CB-28 | 54.6 | Not determined | Not determined |

| | | | |
|---|---|---|---|
| Note: "Not determined" means determination was not carried out. | | | |

As shown in the data in Table 2, anti-human βKlotho antibodies CB-1, CB-6, CB-8, CB-10, CB-16, CB-23, and CB-28 of the present application can specifically bind to human βKlotho. CB-1, CB-6, CB-8, and CB-10 do not bind to human αKlotho, and are highly selective for human βKlotho protein. In addition, some antibodies of the present application, such as CB-10, can bind to mouse βKlotho.

### EXAMPLE 4: Antibody Affinity Determination by Biacore Assay

The binding affinity of antibodies CB-1, CB-6, CB-8 and CB-10 was determined using a Biacore affinity assay. Their binding capabilities to human βKlotho were evaluated by determining the equilibrium dissociation constants (KD) of the purified antibodies. Specifically, by using a His tag capturing agent, an anti-His tag antibody was immobilized in the flow cell of a CM5 chip. Human βKlotho extracellular segment proteins (~80RU) were captured on the flow cells. The purified antibodies (formulated in a 0.005% Tween 2% HEPES buffer) were subsequently injected at a flow rate of 30 L/minute and the binding kinetics was evaluated at 25°C. The results are shown below.

**Table 3: Antibody Affinity Measurements**

| Sample | Ka (1/Ms) | Kd (1/s) | KD (M) |
|---|---|---|---|
| CB-1 | 4.88E+04 | 3.10E-04 | 6.36E-09 |
| CB-6 | 5.61E+05 | 2.82E-04 | 5.01E-10 |
| CB-8 | 6.50E+04 | 5.44E-04 | 8.37E-09 |
| CB-10 | 5.91E+06 | 9.80E-03 | 1.66E-09 |

As shown in the data in Table 3, the anti-human βKlotho antibodies of the present application, such as CB-1, CB-6, CB-8 and CB-10, have strong binding affinity with human βKlotho.

### EXAMPLE 5: Antibody Affinity Maturation Modification

Affinity maturation modifications were carried out on the anti-human βKlotho antibodies of the present application using the phage display method. In the following, antibody CB-8 is used as an example to describe the affinity maturation method.

The genetic sequence of the variable region of antibody CB-8 was constructed into a phage plasmid vector, and the following four saturated mutant libraries were constructed with the phage plasmid vector as a template: 1) a saturated mutant library comprising single-point mutation combinations having a random mutant amino acid in each of light chain CDR1, light chain CDR3, and heavy chain CDR3; 2) a saturated mutant library comprising single-point mutation combinations having a random mutant amino acid in each of in light chain CDR2, heavy chain CDR1, and heavy chain CDR2; 3) a saturated mutation library comprising two-point mutation combinations having random mutations of two adjacent amino acids in light chain CDR3; and 4) a saturated mutation library comprising two-point mutation combinations having random mutations of two adjacent amino acids in heavy chain CDR3. The plasmids in the four saturated mutation libraries were constructed into phage libraries that meet the storage capacity requirements, and the diversity of the phage libraries was determined by sequencing.

A human βKlotho extracellular segment protein (Kactus Biosystems) was used to pan phages from the libraries. The human βKlotho extracellular segment protein was coated onto an immune tube, which was then blocked with a 5% milk PBS blocking solution. Phage at an appropriate concentration was added to the immune tube for incubation for 2 hours at room temperature. The immune tube was washed several times with PBST and then three times with PBS. Finally, the phages bound to the human βKlotho extracellular segment protein were eluted with 0.25% trypsin and collected. The collected phages were used to infect *E.coli* SS320, thereby amplifying the phages. The amplified phages were collected and panned again using the above method. A total of three rounds of panning were conducted.

Monoclonal clones were selected from the libraries after panning for amplification, and positive clones that bound to human βKlotho protein were identified using an ELISA method. These positive clones were then sequenced. The method for determining the binding of the phages to human βKlotho protein by ELISA is decribed below. Human βKlotho protein was coated onto a 96-well plate and the plate was then blocked with a 5% milk PBS blocking solution. Phage supernatants were added for incubation at room temperature for one hour. The plate was washed with PBST and then incubated with a HRP-labeled anti-M13 antibody for one hour. After several washes, a TMB substrate solution was added for incubation at room temperature for 5-10 minutes. Finally, 2 M H₂SO₄ was added to eliminate the reaction, and absorbance was read at 450 nm.

The titers of positive clones were determined, followed by detection of the binding of the phages with different titers to human βKlotho protein using an ELISA assay. The affinity of the positive antibodies was ranked according to a fitted binding curve. Based on the ranking results, the variable region sequences of the top 8 antibodies in terms of binding affinity were selected. The sequences are shown in the following table.

Eight antibody CB-8-derived antibody variable region sequences and the original CB-8 sequence were used to prepare full-length antibodies utilizing the human heavy chain IgG1 constant region and the human light chain kappa constant region. The CDR region and variable region sequences of the present application are as follows:

**Table 4: Antibody Variable Region Sequences**

| **Antibody** | **Numbering Scheme** | **VH** | | | **VL** | | |
|---|---|---|---|---|---|---|---|
| | | **CDR1** | **CDR2** | **CDR3** | **CDR1** | **CDR2** | **CDR3** |
| CB-8 | Kabat | SYGIS SEQ ID NO. 1 | WISAYNGNTNYAQKLQG SEQ ID NO.2 | LYGSGSLYY SEQ ID NO.3 | QASQDIRNYIN SEQ ID NO.4 | AASSLQS SEQ NO.5 ID | QQSYSTPQWT SEQ ID NO.6 |
| | IMGT | GYTFTSYG SEQ ID NO. 7 | ISAYNGNT SEQ ID NO. 8 | ATLYGSGSLYY SEQ ID NO. 9 | QDIRNY SEQ ID NO.10 | AAS SEQ ID NO.11 | QQSYSTPQWT SEQ ID NO.6 |
| VH: | | | | | | | |
| EVQLVQSGAEVKKPGASVKVSCKASGYTFTSYGISWVRQAPGQGLEWMGWISAYNGNTNYAQKLQGRVTMTTDTSTSTAYMELRSLRSD DTAVYYCATLYGSGSLYYWGQGTLVTVSS | | | | | | | |
| SEQ ID NO. 12 | | | | | | | |
| VL: | | | | | | | |
| DIVMTQSPSSLSAPVGDRVTITCQASQDIRNYINWFQQKPGKAPKLLIYAASSLQSGVPSRFSGSGSGTDFTLTISSLQPEDFATYYCQQSYSTP QWTFGQGTKVEIK | | | | | | | |
| SEQ ID NO.13 | | | | | | | |
| CB-8-3 | Kabat | SYGIS SEQ ID NO.1 | WISAYNGRTNYAQKLQG SEQ ID NO.14 | LYGSGSLYY SEQ ID NO.3 | QASQDIRNYIN SEQ ID NO.4 | AASRLQS SEQ ID NO. 15 | QQSYSTPQWT SEQ ID NO.6 |
| | IMGT | GYRFTSYG SEQ ID NO.16 | ISAYNGRT SEQ ID NO.17 | ATLYGSGSLYY SEQ ID NO.9 | QDIRNY SEQ ID NO.10 | AAS SEQ ID NO.11 | QQSYSTPQWT SEQ ID NO.6 |
| VH: | | | | | | | |
| EVQLVQSGAEVKKPGASVKVSCKASGYRFTSYGISWVRQAPGQGLEWMGWISAYNGRTNYAQKLQGRVTMTTDTSTSTAYMELRSLRSD DTAVYYCATLYGSGSLYYWGQGTLVTVSS | | | | | | | |
| SEQ ID NO.18 | | | | | | | |
| VL: | | | | | | | |
| DIVMTQSPSSLSAPVGDRVTITCQASQDIRNYINWFQQKPGKAPKLLIYAASRLQSGVPSRFSGSGSGTDFTLTISSLQPEDFATYYCQQSYST PQWTFGQGTKVEIK | | | | | | | |
| SEQ ID NO.19 | | | | | | | |
| CB-8-14 | Kabat | SYGIS | WISAYTGNTNYAQKLQG | LYGSGSLYY | QASQDIRNYIN | AASSRQS | QQSYSTPQWT |
| | | SEQ ID NO.1 | SEQ ID NO.20 | SEQ ID NO.3 | SEQ ID NO.4 | SEQ ID NO.21 | SEQ ID NO.6 |
| | IMGT | GYTFRSYG SEQ ID NO.22 | ISAYTGNT SEQ ID NO.23 | ATLYGSGSLYY SEQ ID NO.9 | QDIRNY SEQ ID NO.10 | AAS SEQ ID NO.11 | QQSYSTPQWT SEQ ID NO.6 |
| VH: | | | | | | | |
| EVQLVQSGAEVKKPGASVKVSCKASGYTFRSYGISWVRQAPGQGLEWMGWISAYTGNTNYAQKLQGRVTMTTDTSTSTAYMELRSLRSD DTAVYYCATLYGSGSLYYWGQGTLVTVSS | | | | | | | |
| SEQ ID NO.24 | | | | | | | |
| VL: | | | | | | | |
| DIVMTQSPSSLSAPVGDRVTITCQASQDIRNYINWFQQKPGKAPKLLIYAASSRQSGVPSRFSGSGSGTDFTLTISSLQPEDFATYYCQQSYST PQWTFGQGTKVEIK | | | | | | | |
| SEQ ID NO.25 | | | | | | | |
| CB-8-23 | Kabat | SGGIS SEQ ID NO.26 | WISAYNGRTNYAQKLQG SEQ ID NO.14 | LYGSGSLYY SEQ ID NO.3 | QASQDIRNYIN SEQ ID NO.4 | AASFLQS SEQ ID NO.27 | QQSYSTPQWT SEQ ID NO.6 |
| | IMGT | GYTFTSGG SEQ ID NO.28 | ISAYNGRT SEQ ID NO.17 | ATLYGSGSLYY SEQ ID NO.9 | QDIRNY SEQ ID NO.10 | AAS SEQ ID NO.11 | QQSYSTPQWT SEQ ID NO.6 |
| VH: | | | | | | | |
| EVQLVQSGAEVKKPGASVKVSCKASGYTFTSGGISWVRQAPGQGLEWMGWISAYNGRTNYAQKLQGRVTMTTDTSTSTAYMELRSLRSD DTAVYYCATLYGSGSLYYWGQGTLVTVSS | | | | | | | |
| SEQ ID NO.29 | | | | | | | |
| VL: | | | | | | | |
| DIVMTQSPSSLSAPVGDRVTITCQASQDIRNYINWFQQKPGKAPKLLIYAASFLQSGVPSRFSGSGSGTDFTLTISSLQPEDFATYYCQQSYSTP QWTFGQGTKVEIK | | | | | | | |
| SEQ ID NO.30 | | | | | | | |
| CB-8-24 | Kabat | SYGIS SEQ ID NO.1 | WISAYNGNTNYAQKLQG SEQ ID NO.2 | LLPSGSLYY SEQ ID NO.31 | QASQDIRNYIN SEQ ID NO.4 | AASSLQS SEQ ID NO. 5 | QQSYSTPQWT SEQ ID NO.6 |
| | IMGT | GYTFTSYG SEQ ID NO.7 | ISAYNGNT SEQ ID NO. 8 | ATLLPSGSLYY SEQ ID NO.32 | QDIRNY SEQ ID NO.10 | AAS SEQ ID NO.11 | QQSYSTPQWT SEQ ID NO.6 |
| VH: | | | | | | | |
| EVQLVQSGAEVKKPGASVKVSCKASGYTFTSYGISWVRQAPGQGLEWMGWISAYNGNTNYAQKLQGRVTMTTDTSTSTAYMELRSLRSD DTAVYYCATLLPSGSLYYWGQGTLVTVSS | | | | | | | |
| SEQ ID NO.33 | | | | | | | |
| VL: | | | | | | | |
| DIVMTQSPSSLSAPVGDRVTITCQASQDIRNYINWFQQKPGKAPKLLIYAASSLQSGVPSRFSGSGSGTDFTLTISSLQPEDFATYYCQQSYSTP QWTFGQGTKVEIK | | | | | | | |
| SEQ ID NO.13 | | | | | | | |
| SEQ ID NO.13 CB-8-26 | Kabat | SYGIS SEQ ID NO. 1 | WISAYNGRTNYAQKLQG SEQ ID NO.14 | LYGSGSLYY SEQ ID NO.3 | QASQDIRNYIN SEQ ID NO.4 | AASGLQS SEQ ID NO.34 | QQSYSTPQWT SEQ ID NO.6 |
| | IMGT | GYTFKSYG SEQ ID NO.35 | ISAYNGRT SEQ ID NO.17 | ATLYGSGSLYY SEQ ID NO.9 | QDIRNY SEQ ID NO.10 | AAS SEQ ID NO.11 | QQSYSTPQWT SEQ ID NO.6 |
| VH: | | | | | | | |
| EVQLVQSGAEVKKPGASVKVSCKASGYTFKSYGISWVRQAPGQGLEWMGWISAYNGRTNYAQKLQGRVTMTTDTSTSTAYMELRSLRSD DTAVYYCATLYGSGSLYYWGQGTLVTVSS | | | | | | | |
| SEQ ID NO.36 | | | | | | | |
| VL: | | | | | | | |
| DIVMTQSPSSLSAPVGDRVTITCQASQDIRNYINWFQQKPGKAPKLLIYAASGLQSGVPSRFSGSGSGTDFTLTISSLQPEDFATYYCQQSYST PQWTFGQGTKVEIK | | | | | | | |
| SEQ ID NO.37 | | | | | | | |
| CB-8-37 | Kabat | SYGIS SEQ ID NO.1 | WISAYSGNTNYAQKLQG SEQ ID NO.38 | LYGSGSLYY SEQ ID NO.3 | QASQDIRNYIN SEQ ID NO.4 | AASFLQS SEQ ID NO.27 | QQSYSTPQWT SEQ ID NO.6 |
| | IMGT | GYAFTSYG SEQ ID NO.39 | ISAYSGNT SEQ ID NO.40 | ATLYGSGSLYY SEQ ID NO.9 | QDIRNY SEQ ID NO.10 | AAS SEQ ID NO.11 | QQSYSTPQWT SEQ ID NO.6 |
| VH: | | | | | | | |
| EVQLVQSGAEVKKPGASVKVSCKASGYAFTSYGISWVRQAPGQGLEWMGWISAYSGNTNYAQKLQGRVTMTTDTSTSTAYMELRSLRSD DTAVYYCATLYGSGSLYYWGQGTLVTVSS | | | | | | | |
| SEQ ID NO.41 | | | | | | | |
| VL: | | | | | | | |
| DIVMTQSPSSLSAPVGDRVTITCQASQDIRNYINWFQQKPGKAPKLLIYAASFLQSGVPSRFSGSGSGTDFTLTISSLQPEDFATYYCQQSYSTP QWTFGQGTKVEIK | | | | | | | |
| SEQ ID NO.30 | | | | | | | |
| CB-8-40 | Kabat | SYGIS SEQ ID NO. 1 | WISAYSGNTNYAQKLQG SEQ ID NO.38 | LYGSGSLYY SEQ ID NO.3 | QASQDIRNYIN SEQ ID NO.4 | AASYLQS ID SEQ NO.42 | QQSYSTPQWT SEQ ID NO.6 |
| | IMGT | GYTFTSYG SEQ ID NO.7 | ISAYSGNT SEQ ID NO.40 | ATLYGSGSLYY SEQ ID NO. 9 | QDIRNY SEQ ID NO.10 | AAS SEQ ID NO.11 | QQSYSTPQWT SEQ ID NO.6 |
| VH: | | | | | | | |
| EVQLVQSGAEVKKPGASVKVSCKASGYTFTSYGISWVRQAPGQGLEWMGWISAYSGNTNYAQKLQGRVTMTTDTSTSTAYMELRSLRSD DTAVYYCATLYGSGSLYYWGQGTLVTVSS | | | | | | | |
| SEQ ID NO.43 | | | | | | | |
| VL: | | | | | | | |
| DIVMTQSPSSLSAPVGDRVTITCQASQDIRNYINWFQQKPGKAPKLLIYAASYLQSGVPSRFSGSGSGTDFTLTISSLQPEDFATYYCQQSYST PQWTFGQGTKVEIK | | | | | | | |
| SEQ ID NO.44 | | | | | | | |
| SEQ CB-8-41 | Kabat | SYGIS SEQ ID NO. 1 | WISAYTGNTNYAQKLQG SEQ ID NO.20 | LYGSGSLYY SEQ ID NO.3 | QASQDIRNYIN SEQ ID NO.4 | AASHLQS SEQ ID NO.45 | QQSYSTPQWT SEQ ID NO.6 |
| | IMGT | GYLFTSYG SEQ ID NO.46 | ISAYTGNT SEQ ID NO.23 | ATLYGSGSLYY SEQ ID NO. 9 | QDIRNY SEQ ID NO.10 | AAS SEQ ID NO.11 | QQSYSTPQWT SEQ ID NO.6 |
| VH: | | | | | | | |
| EVQLVQSGAEVKKPGASVKVSCKASGYLFTSYGISWVRQAPGQGLEWMGWISAYTGNTNYAQKLQGRVTMTTDTSTSTAYMELRSLRSD DTAVYYCATLYGSGSLYYWGQGTLVTVSS | | | | | | | |
| SEQ ID NO.47 | | | | | | | |
| VL: | | | | | | | |
| DIVMTQSPSSLSAPVGDRVTITCQASQDIRNYINWFQQKPGKAPKLLIYAASHLQSGVPSRFSGSGSGTDFTLTISSLQPEDFATYYCQQSYST PQWTFGQGTKVEIK | | | | | | | |
| SEQ ID NO.48 | | | | | | | |

The antibodies described above were tested for affinity using Gator (Gator bio). Briefly, an antibody was first captured using a probe coated with Protein A (Gator bio). After the baseline was monitored for 60 seconds, the antibody-to-the antigen binding was determined with the human βKlotho extracellular segment protein described above at varying concentrations (400nM-50nM). Then, the dissociation was detected in a K buffer (Gator bio). Meanwhile, the function of an antibody to activate the human or cynomolgus monkey FGFR1c/βKlotho receptor was detected using the reporter-based assay described in Example 2. The results are shown in Table 5.

**Table 5: Antibody Affinity Test Results**

| Sample | Gator affinity determination | | | EC₅₀ for antibody-induced cell activation, as determined by the reporter-based assay | |
|---|---|---|---|---|---|
| | Ka (1/Ms) | Kd (1/s) | KD (M) | Human βKlotho/FGFR1c (nM) | Cynomolgus monkey βKlotho/FGFR1c (nM) |
| CB-8 | 1.60E+03 | 6.21E-04 | 3.88E-07 | 3.74 | 2.86 |
| CB-8-3 | 3.64E+04 | 1.33E-04 | 3.64E-09 | 1.39 | 0.88 |
| CB-8-14 | 1.96E+04 | 9.72E-05 | 4.96E-09 | 0.95 | 1.08 |
| CB-8-23 | 2.45E+04 | 1.73E-04 | 7.08E-09 | 0.57 | 0.55 |
| CB-8-24 | 7.25E+03 | 4.57E-04 | 6.31E-08 | 0.68 | 0.15 |
| CB-8-26 | 1.52E+04 | 1.32E-04 | 8.70E-09 | 0.66 | 0.71 |
| CB-8-37 | 1.99E+04 | 6.29E-05 | 3.16E-09 | 0.92 | 0.54 |
| CB-8-40 | 3.24E+04 | 4.06E-05 | 1.26E-09 | 0.89 | 0.49 |
| CB-8-41 | 2.70E+04 | 2.07E-05 | 7.67E-10 | 1.43 | 1.57 |

As shown in Table 5, after the affinity maturation modification, the binding affinity of all the modified antibodies to human βKlotho, human βKlotho/FGFR1c, and cynomolgus monkey βKlotho/FGFR1c were significantly improved compared with antibody CB-8 before the modification.

### EXAMPLE 6: Potential PTM Site Modification

In the present application, the CDRs were further analyzed in detail for the antibodies after the affinity maturation modification. In the following, CB-8-24 is used as an example to describe the method of potential PTM site modification.

Through the sequence analysis, it was found that a potential deamidation site NG was present in the heavy chain CDR2 of CB-8-24, while the corresponding site in CB-8-40 was SG, which can avoid potential deamidation issues. For example, a new antibody sequence CB-8-42 was designed in view of the sequences of CB-8-24 and CB-8-40. The human IgG1 (E233A/L235A) heavy chain constant region sequence and the human kappa constant region sequence were used to prepare a full-length antibody containing the VH and VL of CB-8-42.

| | | | | | | | |
|---|---|---|---|---|---|---|---|
| CB-8-42 | Kabat | SYGIS SEQ ID NO.1 | WISAYSGNTNYAQKLQG SEQ ID NO.38 | LLPSGSLYY SEQ ID NO.31 | QASQDIRNYIN SEQ ID NO.4 | AASYLQS SEQ ID NO.42 | QQSYSTPQWT SEQ ID NO.6 |
| | IMGT | GYTFTSYG SEQ ID NO.7 | ISAYSGNT SEQ ID NO.40 | ATLLPSGSLYY SEQ ID NO.32 | QDIRNY SEQ ID NO.10 | AAS SEQ ID NO. 11 | QQSYSTPQWT SEQ ID NO.6 |
| VH: EVQLVQSGAEVKKPGASVKVSCKASGYTFTSYGISWVRQAPGQGLEWMGWISAYSGNTNYAQKLQGRVTMTTDTSTSTAYMELRSLRSD DTAVYYCATLLPSGSLYYWGQGTLVTVSS SEQ ID NO.49 | | | | | | | |
| VL: DIVMTQSPSSLSAPVGDRVTITCQASQDIRNYINWFQQKPGKAPKLLIYAASYLQSGVPSRFSGSGSGTDFTLTISSLQPEDFATYYCQQSYST PQWTFGQGTKVEIK SEQ ID NO.44 | | | | | | | |

Constant region sequences are shown below.
Heavy chain constant region sequence (HC): 


SEQ ID NO.51
Light chain constant region sequence (LC):

Light chain constant region sequence (LC):



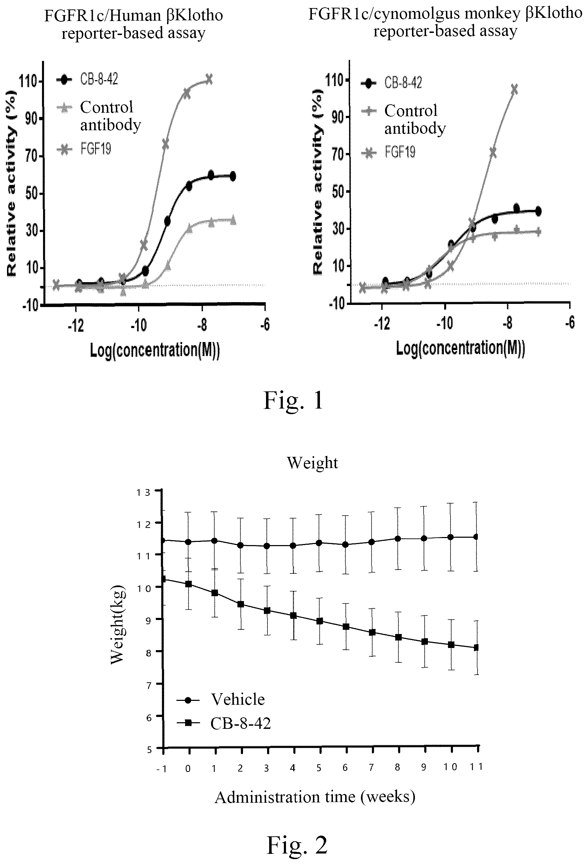

The function of full-length antibody CB-8-42 to activate the human or monkey FGFR1c/βKlotho receptor was detected using the reporter-based assay as described in Example 2. The results (see Table 6) showed that the activating effect of CB-8-42 on the FGFR1c/βKlotho receptor was significantly stronger than that of the control antibody. Particularly, the maximal cellular response of CB-8-42 was higher than that of the control antibody. Results are shown in Fig. 1. At the same time, the function of antibodies to activate FGFR2c/βKlotho, FGFR3c/βKlotho, or FGFR4/βKlotho was detected using the same method. The results showed that CB-8-42 could not activate FGFR2c/βKlotho, FGFR3c/βKlotho, or FGFR4/βKlotho receptor, showing its excellent specificity for human FGFR1c/βKlohto.

**Table 6: Antibody Activation Experimental Results**

| **Sample** | **Human FGFR1c/ βKlohto EC₅₀ (nM)** | **Cynomolgus monkey FGFR1c/ βKlohto EC₅₀ (nM)** | **Human FGFR2c/ βKlohto EC₅₀ (nM)** | **Human FGFR3c/ βKlohto EC₅₀ (nM)** | **Human FGFR4/ βKlohto EC₅₀ (nM)** |
|---|---|---|---|---|---|
| FGF19 | 0.41 | 2.23 | 0.27 | 0.30 | 0.05 |
| Control antibody | 1.20 | 0.064 | >100 | >100 | >100 |
| CB-8-42 | 0.66 | 0.17 | >100 | >100 | >100 |

In view of the above results, the anti-human βKlotho antibodies of the present application, particularly CB-8-42, demonstrate superior performance as compared with the control antibody (5H23, the sequence of which is derived from antibody 5H23 described in NGM patent CN 201580016315.8) in terms of binding ability, or binding specificity, or receptor activation ability. For example, as shown in Fig.1 and Table 6, the ability of CB-8-42 to activate human FGFR1c/βKlohto is nearly twice that of the control antibody, and its maximal relative activity is significantly better than that of the control antibody (Fig.1).

### EXAMPLE 7: In Vivo Pharmacodynamics Study in Animals

In this Emample, the *in vivo* efficacy of an anti-human βKlotho antibody was evaluated in an animal *in vivo* efficacy evaluation model (using cynomolgus monkey as an example).

It was studied in the present application the potential metabolism improving effect of an anti-human βKlotho antibody (using CB-8-42 as an example) in an obese cynomolgus monkey model. The *in vivo* efficacy of the antibody of the present application was assessed by monitoring changes in ingestion, body weight, BMI, and biochemical indicators (fasting blood glucose, insulin, triglycerides, cholesterol, low density lipoprotein, high density lipoprotein, liver fat content, and so on) in cynomolgus monkeys after injection of the antibody.

In the actual study, ten male obese cynomolgus monkeys (older than 11 years, weighing more than 8.5 kg and tested for an MRI liver fat content ≥8%) were selected. According to the body weight and liver fat content, the animals were randomly assigned to the vehicle group (n=5) and the antibody group (n=5) which were subjected to two-week acclimatization. During this period, clinical observations and food intake statistics were performed daily, and body weight measurements and BMI calculations were performed weekly. At the same time, blood biochemical basic indexes, including fasting blood glucose, insulin, triglycerides, cholesterol, low-density lipoprotein, and high-density lipoprotein, were detected before administration. Animals were injected subcutaneously with a vehicle (a 20 mM histidine buffer, 4.5% sorbitol, pH 5.7) or the anti-βKlotho antibody (CB-8-42, 10 mg/kg) on Days 1, 29, and 57, following the 2-week acclimatization period. Clinical observations and food intake statistics were performed daily, and body weight was measured weekly during the study. Three weeks (Day 22), seven weeks (Day 50), and eleven weeks (Day 78) after the first dose, blood samples were taken from the animals after an overnight fast, and serum glucose, insulin, triglycerides, cholesterol, low-density lipoprotein, and high-density lipoprotein levels were determined. Liver fat contents were determined using MRI at 6 and 12 weeks after the first dose.

### Weight

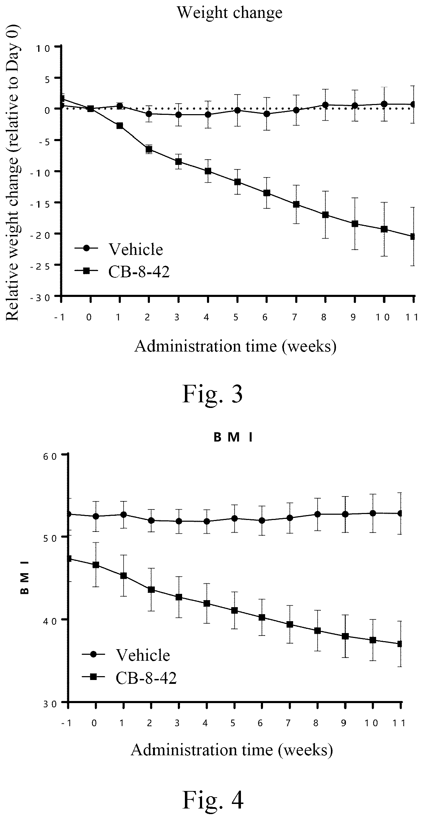

As shown in FIG. 2-4, there was no significant change in the body weight of the vehicle-treated animals. In contrast, the body weight of the anti-human βKlotho antibody CB-8-42-treated animals gradually decreased. By the eleventh week after administration, the body weight of the CB-8-42-treated animals decreased by an average of 20%. Accordingly, there were no significant change in the BMIs of the vehicle-treated animals, while the BMIs in the anti-human βKlotho antibody CB-8-42-treated animals decreased by an average of 20%.

### Food intake

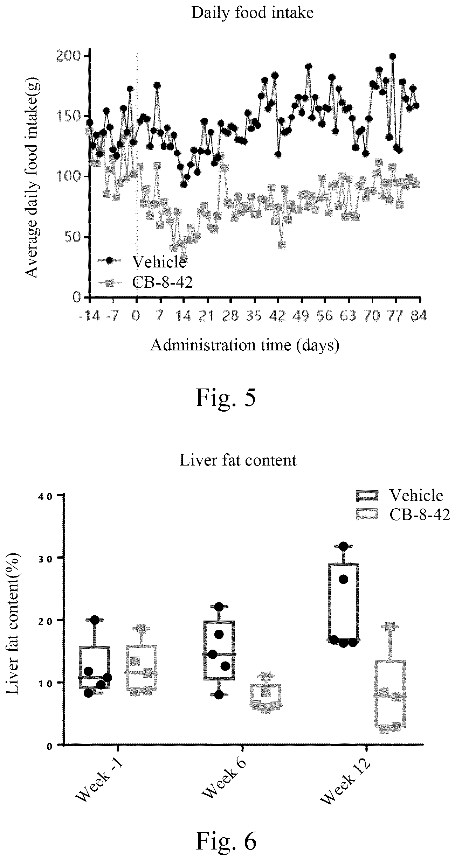

As shown in Fig.5, food intake of the vehicle-treated animals substantively remained stable throughout the experiment, and the food intake was approximately 140 g/day. Food intake in animals treated with the anti-human βKlotho antibody CB-8-42 was significantly reduced after the treatment. In the acclimatization phase, the average food intake was about 112 g/day, and in the antibody treatment phase, the food intake decreased to an average of about 78 g/day, decreased by about 30%.

### Liver fat content

As shown in Fig.6, liver fat content gradually increased in the vehicle-treated animals, and there was a statistical difference in liver fat content twelve weeks after the treatment as compared with the pre-administration content (p<0.05). After treatment with the anti-human βKlotho antibody CB-8-42, the mean liver fat content in animals decreased from 12.1% before administration to 7.5% six weeks after the first dose, and maintained at 8.1% twelve weeks after the treatment. The liver fat content of the antibody group was comparable to that of the vehicle group before administration, both at 12.1%. Twelve weeks after receiving the anti-human βKlotho antibody CB-8-42 treatment, the liver fat content in the antibody group was significantly lower than that in the vehicle group (8.1% vs. 21.6%, p<0.01).

### Fasting blood glucose content

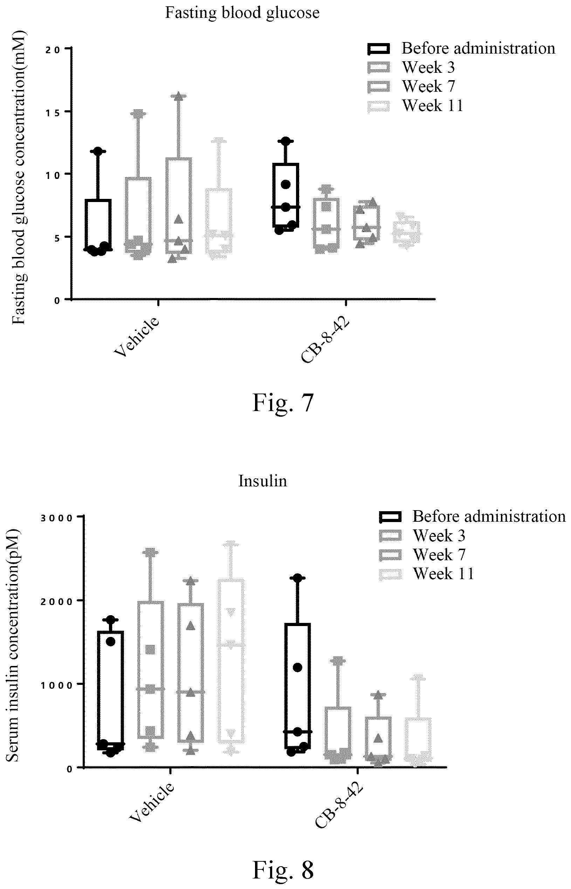

As shown in Fig.7, fasting blood glucose in the vehicle-treated animals slowly increased during the experiment, while fasting blood glucose in the animals significantly decreased after treatment with the anti-human βKlotho antibody CB-8-42.

### Serum insulin level

As shown in Fig.8, serum insulin level of the vehicle-treated animals significantly increased during the experiment, while the serum insulin level of the animals significantly decreased after treatment with the anti-human βKlotho antibody CB-8-42.

### Serum triglyceride level

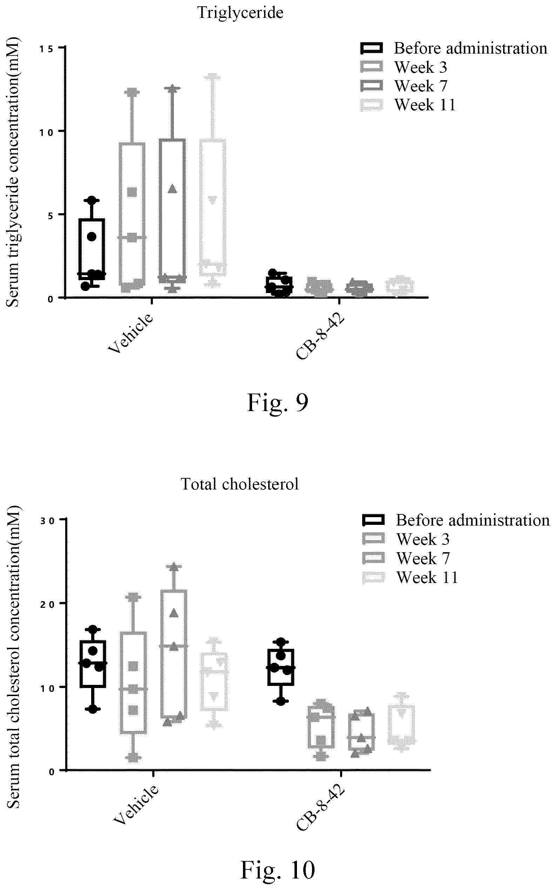

As shown in Fig.9, serum triglyceride level in animals treated with the vehicle increased significantly during the experiment, while serum triglyceride level in animals remained stable after treatment with anti-human βKlotho antibody CB-8-42.

### Serum total cholesterol level

As shown in Fig.10, serum total cholesterol level of the vehicle-treated animals remained stable during the experiment, while the serum total cholesterol level of the animals significantly decreased after the treatment with the anti-human βKlotho antibody CB-8-42.

### Serum low density lipoprotein level

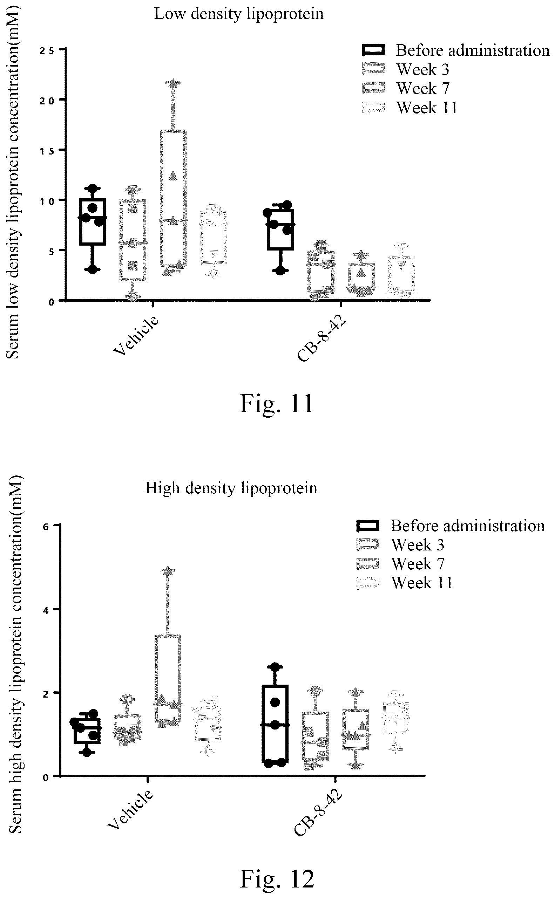

As shown in Fig.11, serum low density lipoprotein level of the vehicle-treated animals remained basically stable during the experiment, while the serum low density lipoprotein level of the animals significantly decreased after treatment with the anti-human βKlotho antibody CB-8-42.

### Serum high density lipoprotein level

As shown in Fig.12, serum high-density lipoprotein level of the animals receiving the vehicle and the anti-human βKlotho antibody CB-8-42 remained stable during the experiment.

According to the above experimental results, compared with the vehicle group, the administration of the anti-βKlotho antibody of the present application can significantly improve the metabolic level of cynomolgus monkeys. Changes in ingestion, body weight, BMI, and a variety of biochemical indicators such as fasting blood glucose, insulin, triglycerides, cholesterol, low-density lipoprotein, high-density lipoprotein, liver fat content and so on in cynomolgus monkeys demonstrate the great potential and potential clinical efficacy of the anti-human βKlotho antibody of the present application in the treatment or alleviation of diabetes, dyslipidemia, non-alcohol steatohepatitis (NASH), non-alcohol steatohepatitis (NAFLD), cardiovascular disease, metabolic syndrome or obesity. In general, the results from the *in vivo* pharmacodynamic experiments in animals show that the anti-human βKlotho antibody of the present application had significantly alleviating effects for the diseases described above in the obese cynomolgus monkey model, and had good *in vivo* safety.

The use of any and all embodiments or exemplary language (e.g., "such as") provided herein is intended only to better illustrate the inventions of the present application and does not limit the scope of the inventions unless otherwise required. The language in the specification should not be construed as indicating that any element not recited in a claim is necessary for the implementation of the inventions of the present application.

All publications and patent applications cited in the present specification are incorporated herein by reference as if each individual publication or patent application were specifically and individually identified by reference. Furthermore, any theory, mechanism, proof, or discovery described herein is intended to further facilitate the understanding of the present application and is not intended to limit the present application in any way to such theory, mechanism, proof, or discovery. Although the present application has been shown and described in detail in the drawings and the foregoing description, the present application should be considered illustrative and not limiting.

## Claims

1. An anti-βKlotho antibody or an antigen-binding portion thereof, comprising a heavy chain variable region and a light chain variable region, wherein the heavy chain variable region comprises a heavy chain CDR1 (HCDR1), a heavy chain CDR2 (HCDR2) and a heavy chain CDR3 (HCDR3), and the light chain variable region comprises a light chain CDR1 (LCDR1), a light chain CDR2 (LCDR2) and a light chain CDR3 (LCDR3), and wherein:
(1) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.2 or has at least 80%, 85%, 90%, 95% or 99% identity to the sequence as set forth in SEQ ID NO.2, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.5 or has at least 80%, 85%, 90%, 95% or 99% identity to the sequence as set forth in SEQ ID NO.5, and the sequence of the LCDR3 is the sequence as set forth in SEQ ID NO.6 or has at least 80%, 85%, 90%, 95% or 99% identity to the sequence as set forth in SEQ ID NO.6; or
(2) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO. 14 or has at least 80%, 85%, 90%, 95% or 99% identity to the sequence as set forth in SEQ ID NO. 14, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO. 15 or has at least 80%, 85%, 90%, 95% or 99% identity to the sequence as set forth in SEQ ID NO. 15, and the sequence of the LCDR3 is the sequence as set forth in SEQ ID NO.6 or has at least 80%, 85%, 90%, 95% or 99% identity to the sequence as set forth in SEQ ID NO.6; or
(3) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.20 or has at least 80%, 85%, 90%, 95% or 99% identity to the sequence as set forth in SEQ ID NO.20, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.21 or has at least 80%, 85%, 90%, 95% or 99% identity to the sequence as set forth in SEQ ID NO.21, and the sequence of the LCDR3 is the sequence as set forth in SEQ ID NO.6 or has at least 80%, 85%, 90%, 95% or 99% identity to the sequence as set forth in SEQ ID NO.6; or
(4) the sequence of the HCDR1 is the sequence as set forth in SEQ ID NO.26 or has at least 80%, 85%, 90%, 95% or 99% identity to the sequence as set forth in SEQ ID NO.26, the sequence of the HCDR2 is the sequence as set forth in SEQ ID NO. 14 or has at least 80%, 85%, 90%, 95% or 99% identity to the sequence as set forth in SEQ ID NO. 14, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.27 or has at least 80%, 85%, 90%, 95% or 99% identity to the sequence as set forth in SEQ ID NO.27, and the sequence of the LCDR3 is the sequence as set forth in SEQ ID NO.6 or has at least 80%, 85%, 90%, 95% or 99% identity to the sequence as set forth in SEQ ID NO.6; or
(5) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.2 or has at least 80%, 85%, 90%, 95% or 99% identity to the sequence as set forth in SEQ ID NO.2, the sequence of the HCDR3 is the sequence as set forth in SEQ ID NO.31 or has at least 80%, 85%, 90%, 95% or 99% identity to the sequence as set forth in SEQ ID NO.31, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.5 or has at least 80%, 85%, 90%, 95% or 99% identity to the sequence as set forth in SEQ ID NO.5, and the sequence of the LCDR3 is the sequence as set forth in SEQ ID NO.6 or has at least 80%, 85%, 90%, 95% or 99% identity to the sequence as set forth in SEQ ID NO.6; or
(6) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO. 14 or has at least 80%, 85%, 90%, 95% or 99% identity to the sequence as set forth in SEQ ID NO.14, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.34 or has at least 80%, 85%, 90%, 95% or 99% identity to the sequence as set forth in SEQ ID NO.34, and the sequence of the LCDR3 is the sequence as set forth in SEQ ID NO.6 or has at least 80%, 85%, 90%, 95% or 99% identity to the sequence as set forth in SEQ ID NO.6; or
(7) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.27 or has at least 80%, 85%, 90%, 95% or 99% identity to the sequence as set forth in SEQ ID NO.27, and the sequence of the LCDR3 is the sequence as set forth in SEQ ID NO.6 or has at least 80%, 85%, 90%, 95% or 99% identity to the sequence as set forth in SEQ ID NO.6; or
(8) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.42 or has at least 80%, 85%, 90%, 95% or 99% identity to the sequence as set forth in SEQ ID NO.42, and the sequence of the LCDR3 is the sequence as set forth in SEQ ID NO.6 or has at least 80%, 85%, 90%, 95% or 99% identity to the sequence as set forth in SEQ ID NO.6; or
(9) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.20 or has at least 80%, 85%, 90%, 95% or 99% identity to the sequence as set forth in SEQ ID NO.20, the sequence of the HCDR3 is the sequence as set forth in SEQ ID NO.3 or has at least 80%, 85%, 90%, 95% or 99% identity to the sequence as set forth in SEQ ID NO.3, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.45 or has at least 80%, 85%, 90%, 95% or 99% identity to the sequence as set forth in SEQ ID NO.45, and the sequence of the LCDR3 is the sequence as set forth in SEQ ID NO.6 or has at least 80%, 85%, 90%, 95% or 99% identity to the sequence as set forth in SEQ ID NO.6; or
(10) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, the sequence of the HCDR3 is the sequence as set forth in SEQ ID NO.31 or has at least 80%, 85%, 90%, 95% or 99% identity to the sequence as set forth in SEQ ID NO.31, the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.42 or has at least 80%, 85%, 90%, 95% or 99% identity to the sequence as set forth in SEQ ID NO.42, and the sequence of the LCDR3 is the sequence as set forth in SEQ ID NO.6 or has at least 80%, 85%, 90%, 95% or 99% identity to the sequence as set forth in SEQ ID NO.6;
wherein the sequences of the HCDRs and LCDRs are defined according to the Kabat definition.

2. An anti-βKlotho antibody or an antigen-binding portion thereof, comprising a heavy chain variable region and a light chain variable region, wherein the heavy chain variable region comprises a heavy chain CDR1 (HCDR1), a heavy chain CDR2 (HCDR2) and a heavy chain CDR3 (HCDR3), and the light chain variable region comprises a light chain CDR1 (LCDR1), a light chain CDR2 (LCDR2) and a light chain CDR3 (LCDR3), and wherein:
(1) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.8 or has at least 80%, 85%, 90%, 95% or 99% identity to the sequence as set forth in SEQ ID NO.8, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO.10 or has at least 80%, 85%, 90%, 95% or 99% identity to the sequence as set forth in SEQ ID NO.10, the sequence of the LCDR2 is the sequence as set forth in SEQ ID NO.11 or has at least 80%, 85%, 90%, 95% or 99% identity to the sequence as set forth in SEQ ID NO.11, and the sequence of the LCDR3 is the sequence as set forth in SEQ ID NO.6 or has at least 80%, 85%, 90%, 95% or 99% identity to the sequence as set forth in SEQ ID NO.6; or
(2) the sequence of the HCDR1 is the sequence as set forth in SEQ ID NO.16 or has at least 80%, 85%, 90%, 95% or 99% identity to the sequence as set forth in SEQ ID NO.16, the sequence of the HCDR2 is the sequence as set forth in SEQ ID NO.17 or has at least 80%, 85%, 90%, 95% or 99% identity to the sequence as set forth in SEQ ID NO.17, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO.10 or has at least 80%, 85%, 90%, 95% or 99% identity to the sequence as set forth in SEQ ID NO.10, the sequence of the LCDR2 is the sequence as set forth in SEQ ID NO.11 or has at least 80%, 85%, 90%, 95% or 99% identity to the sequence as set forth in SEQ ID NO.11, and the sequence of the LCDR3 is the sequence as set forth in SEQ ID NO.6 or has at least 80%, 85%, 90%, 95% or 99% identity to the sequence as set forth in SEQ ID NO.6; or
(3) the sequence of the HCDR1 is the sequence as set forth in SEQ ID NO.22 or has at least 80%, 85%, 90%, 95% or 99% identity to the sequence as set forth in SEQ ID NO.22, the sequence of the HCDR2 is the sequence as set forth in SEQ ID NO.23 or has at least 80%, 85%, 90%, 95% or 99% identity to the sequence as set forth in SEQ ID NO.23, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO.10 or has at least 80%, 85%, 90%, 95% or 99% identity to the sequence as set forth in SEQ ID NO.10, the sequence of the LCDR2 is the sequence as set forth in SEQ ID NO.11 or has at least 80%, 85%, 90%, 95% or 99% identity to the sequence as set forth in SEQ ID NO.11, and the sequence of the LCDR3 is the sequence as set forth in SEQ ID NO.6 or has at least 80%, 85%, 90%, 95% or 99% identity to the sequence as set forth in SEQ ID NO.6; or
(4) the sequence of the HCDR1 is the sequence as set forth in SEQ ID NO.28 or has at least 80%, 85%, 90%, 95% or 99% identity to the sequence as set forth in SEQ ID NO.28, the sequence of the HCDR2 is the sequence as set forth in SEQ ID NO. 17 or has at least 80%, 85%, 90%, 95% or 99% identity to the sequence as set forth in SEQ ID NO. 17, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(5) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.8 or has at least 80%, 85%, 90%, 95% or 99% identity to the sequence as set forth in SEQ ID NO.8, the sequence of the HCDR3 is the sequence as set forth in SEQ ID NO.32 or has at least 80%, 85%, 90%, 95% or 99% identity to the sequence as set forth in SEQ ID NO.32, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(6) the sequence of the HCDR1 is the sequence as set forth in SEQ ID NO.35 or has at least 80%, 85%, 90%, 95% or 99% identity to the sequence as set forth in SEQ ID NO.35, the sequence of the HCDR2 is the sequence as set forth in SEQ ID NO. 17 or has at least 80%, 85%, 90%, 95% or 99% identity to the sequence as set forth in SEQ ID NO. 17, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(7) the sequence of the HCDR1 is the sequence as set forth in SEQ ID NO.39 or has at least 80%, 85%, 90%, 95% or 99% identity to the sequence as set forth in SEQ ID NO.39, the sequence of the HCDR2 is the sequence as set forth in SEQ ID NO.40 or has at least 80%, 85%, 90%, 95% or 99% identity to the sequence as set forth in SEQ ID NO.40, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(8) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.40 or has at least 80%, 85%, 90%, 95% or 99% identity to the sequence as set forth in SEQ ID NO.40, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(9) the sequence of the HCDR1 is the sequence as set forth in SEQ ID NO.46 or has at least 80%, 85%, 90%, 95% or 99% identity to the sequence as set forth in SEQ ID NO.46, the sequence of the HCDR2 is the sequence as set forth in SEQ ID NO.23 or has at least 80%, 85%, 90%, 95% or 99% identity to the sequence as set forth in SEQ ID NO.23, the sequence of the HCDR3 is the sequence as set forth in SEQ ID NO.9 or has at least 80%, 85%, 90%, 95% or 99% identity to the sequence as set forth in SEQ ID NO.9, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO.11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6; or
(10) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.40 or has at least 80%, 85%, 90%, 95% or 99% identity to the sequence as set forth in SEQ ID NO.40, the sequence of the HCDR3 is the sequence as set forth in SEQ ID NO.32 or has at least 80%, 85%, 90%, 95% or 99% identity to the sequence as set forth in SEQ ID NO.32, the sequence of the LCDR1 is the sequence as set forth in SEQ ID NO. 10 or has at least 80%, 85%, 90%, 95% or 99% identity to the sequence as set forth in SEQ ID NO. 10, the sequence of the LCDR2 is the sequence as set forth in SEQ ID NO. 11 or has at least 80%, 85%, 90%, 95% or 99% identity to the sequence as set forth in SEQ ID NO. 11, and the sequence of the LCDR3 is the sequence as set forth in SEQ ID NO.6 or has at least 80%, 85%, 90%, 95% or 99% identity to the sequence as set forth in SEQ ID NO.6;
wherein the sequences of the HCDRs and LCDRs are defined according to the IMGT definition.

3. The anti-βKlotho antibody or antigen-binding portion thereof of claim 1 or 2, wherein the heavy chain variable region has the amino acid sequence as set forth in SEQ ID NO. 12, 18, 24, 29, 33, 36, 41, 43, 47 or 49, or has an amino acid sequence having at least 80%, at least 85%, at least 90%, at least 95% or at least 99% identity to the amino acid sequence as set forth in SEQ ID NO. 12, 18, 24, 29, 33, 36, 41, 43, 47 or 49.

4. The anti-βKlotho antibody or antigen-binding portion thereof of any one of claims 1 to 3, wherein the light chain variable region has the amino acid sequence as set forth in SEQ ID NO. 13, 19, 25, 30, 37, 44 or 48, or has an amino acid sequence having at least 80%, at least 85%, at least 90%, at least 95% or at least 99% identity to the amino acid sequence as set forth in SEQ ID NO. 13, 19, 25, 30, 37, 44 or 48.

5. The anti-βKlotho antibody or antigen-binding portion thereof of any one of claims 1 to 4, wherein
(1) the sequence of the heavy chain variable region is the sequence as set forth in SEQ ID NO. 12, and the sequence of the light chain variable region is the sequence as set forth in SEQ ID NO. 13; or
(2) the sequence of the heavy chain variable region is the sequence as set forth in SEQ ID NO. 18, and the sequence of the light chain variable region is the sequence as set forth in SEQ ID NO. 19; or
(3) the sequence of the heavy chain variable region is the sequence as set forth in SEQ ID NO.24, and the sequence of the light chain variable region is the sequence as set forth in SEQ ID NO.25; or
(4) the sequence of the heavy chain variable region is the sequence as set forth in SEQ ID NO.29, and the sequence of the light chain variable region is the sequence as set forth in SEQ ID NO.30; or
(5) the sequence of the heavy chain variable region is the sequence as set forth in SEQ ID NO.33, and the sequence of the light chain variable region is the sequence as set forth in SEQ ID NO. 13; or
(6) the sequence of the heavy chain variable region is the sequence as set forth in SEQ ID NO.36, and the sequence of the light chain variable region is the sequence as set forth in SEQ ID NO.37; or
(7) the sequence of the heavy chain variable region is the sequence as set forth in SEQ ID NO.41, and the sequence of the light chain variable region is the sequence as set forth in SEQ ID NO.30; or
(8) the sequence of the heavy chain variable region is the sequence as set forth in SEQ ID NO.43, and the sequence of the light chain variable region is the sequence as set forth in SEQ ID NO.44; or
(9) the sequence of the heavy chain variable region is the sequence as set forth in SEQ ID NO.47, and the sequence of the light chain variable region is the sequence as set forth in SEQ ID NO.48; or
(10) the sequence of the heavy chain variable region is the sequence as set forth in SEQ ID NO.49, and the sequence of the light chain variable region is the sequence as set forth in SEQ ID NO.44.

6. An anti-βKlotho antibody or an antigen-binding portion thereof, comprising a heavy chain variable region and a light chain variable region, wherein:
the heavy chain variable region has the amino acid sequence as set forth in SEQ ID NO. 12, 18, 24, 29, 33, 36, 41, 43, 47 or 49, or has an amino acid sequence having at least 80%, at least 85%, at least 90%, at least 95% or at least 99% identity to the amino acid sequence as set forth in SEQ ID NO. 12, 18, 24, 29, 33, 36, 41, 43, 47 or 49; and/or
the light chain variable region has the amino acid sequence as set forth in SEQ ID NO. 13, 19, 25, 30, 37, 44 or 48, or has an amino acid sequence having at least 80%, at least 85%, at least 90%, at least 95% or at least 99% identity to the amino acid sequence as set forth in SEQ ID NO. 13, 19, 25, 30, 37, 44 or 48.

7. An anti-βKlotho antibody or an antigen-binding portion thereof, comprising a heavy chain variable region comprising a heavy chain CDR1 (HCDR1), a heavy chain CDR2 (HCDR2) and a heavy chain CDR3 (HCDR3), wherein:
(1) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.2 or has at least 80%, 85%, 90%, 95% or 99% identity to the sequence as set forth in SEQ ID NO.2, and the sequence of the HCDR3 is the sequence as set forth in SEQ ID NO.3 or has at least 80%, 85%, 90%, 95% or 99% identity to the sequence as set forth in SEQ ID NO.3; or
(2) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO. 14 or has at least 80%, 85%, 90%, 95% or 99% identity to the sequence as set forth in SEQ ID NO. 14, and the sequence of the HCDR3 is the sequence as set forth in SEQ ID NO.3 or has at least 80%, 85%, 90%, 95% or 99% identity to the sequence as set forth in SEQ ID NO.3; or
(3) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.20 or has at least 80%, 85%, 90%, 95% or 99% identity to the sequence as set forth in SEQ ID NO.20, and the sequence of the HCDR3 is the sequence as set forth in SEQ ID NO.3 or has at least 80%, 85%, 90%, 95% or 99% identity to the sequence as set forth in SEQ ID NO.3; or
(4) the sequence of the HCDR1 is the sequence as set forth in SEQ ID NO.26 or has at least 80%, 85%, 90%, 95% or 99% identity to the sequence as set forth in SEQ ID NO.26, the sequence of the HCDR2 is the sequence as set forth in SEQ ID NO. 14 or has at least 80%, 85%, 90%, 95% or 99% identity to the sequence as set forth in SEQ ID NO. 14, and the sequence of the HCDR3 is the sequence as set forth in SEQ ID NO.3 or has at least 80%, 85%, 90%, 95% or 99% identity to the sequence as set forth in SEQ ID NO.3; or
(5) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.2 or has at least 80%, 85%, 90%, 95% or 99% identity to the sequence as set forth in SEQ ID NO.2, and the sequence of the HCDR3 is the sequence as set forth in SEQ ID NO.31 or has at least 80%, 85%, 90%, 95% or 99% identity to the sequence as set forth in SEQ ID NO.31; or
(6) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, and the sequence of the HCDR3 is the sequence as set forth in SEQ ID NO.3 or has at least 80%, 85%, 90%, 95% or 99% identity to the sequence as set forth in SEQ ID NO.3; or
(7) the sequence of the HCDR1 is the sequence as set forth in SEQ ID NO.1 or has at least 80%, 85%, 90%, 95% or 99% identity to the sequence as set forth in SEQ ID NO.1, the sequence of the HCDR2 is the sequence as set forth in SEQ ID NO.38 or has at least 80%, 85%, 90%, 95% or 99% identity to the sequence as set forth in SEQ ID NO.38, and the sequence of the HCDR3 is the sequence as set forth in SEQ ID NO.31 or has at least 80%, 85%, 90%, 95% or 99% identity to the sequence as set forth in SEQ ID NO.31;
wherein the sequences of the HCDRs are defined according to the Kabat definition.

8. An anti-βKlotho antibody or an antigen-binding portion thereof comprising a light chain variable region comprising a light chain CDR1 (LCDR1), a light chain CDR2 (LCDR2) and a light chain CDR3 (LCDR3), wherein:
(1) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.5 or has at least 80%, 85%, 90%, 95% or 99% identity to the sequence as set forth in SEQ ID NO.5, and the sequence of the LCDR3 is the sequence as set forth in SEQ ID NO.6 or has at least 80%, 85%, 90%, 95% or 99% identity to the sequence as set forth in SEQ ID NO.6; or
(2) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.15 or has at least 80%, 85%, 90%, 95% or 99% identity to the sequence as set forth in SEQ ID NO.15, and the sequence of the LCDR3 is the sequence as set forth in SEQ ID NO.6 or has at least 80%, 85%, 90%, 95% or 99% identity to the sequence as set forth in SEQ ID NO.6; or
(3) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.21 or has at least 80%, 85%, 90%, 95% or 99% identity to the sequence as set forth in SEQ ID NO.21, and the sequence of the LCDR3 is the sequence as set forth in SEQ ID NO.6 or has at least 80%, 85%, 90%, 95% or 99% identity to the sequence as set forth in SEQ ID NO.6; or
(4) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.27 or has at least 80%, 85%, 90%, 95% or 99% identity to the sequence as set forth in SEQ ID NO.27, and the sequence of the LCDR3 is the sequence as set forth in SEQ ID NO.6 or has at least 80%, 85%, 90%, 95% or 99% identity to the sequence as set forth in SEQ ID NO.6; or
(5) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.34 or has at least 80%, 85%, 90%, 95% or 99% identity to the sequence as set forth in SEQ ID NO.34, and the sequence of the LCDR3 is the sequence as set forth in SEQ ID NO.6 or has at least 80%, 85%, 90%, 95% or 99% identity to the sequence as set forth in SEQ ID NO.6; or
(6) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.42 or has at least 80%, 85%, 90%, 95% or 99% identity to the sequence as set forth in SEQ ID NO.42, and the sequence of the LCDR3 is the sequence as set forth in SEQ ID NO.6 or has at least 80%, 85%, 90%, 95% or 99% identity to the sequence as set forth in SEQ ID NO.6; or
(7) the sequence of the LCDR1 is the sequence as set forth in SEQ ID NO.4 or has at least 80%, 85%, 90%, 95% or 99% identity to the sequence as set forth in SEQ ID NO.4, the sequence of the LCDR2 is the sequence as set forth in SEQ ID NO.45 or has at least 80%, 85%, 90%, 95% or 99% identity to the sequence as set forth in SEQ ID NO.45, and the sequence of the LCDR3 is the sequence as set forth in SEQ ID NO.6 or has at least 80%, 85%, 90%, 95% or 99% identity to the sequence as set forth in SEQ ID NO.6;
wherein the sequences of the LCDRs are defined according to the Kabat definition.

9. An anti-βKlotho antibody or an antigen-binding portion thereof, comprising a heavy chain variable region comprising a heavy chain CDR1 (HCDR1), a heavy chain CDR2 (HCDR2) and a heavy chain CDR3 (HCDR3), wherein:
(1) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.8 or has at least 80%, 85%, 90%, 95% or 99% identity to the sequence as set forth in SEQ ID NO.8, and the sequence of the HCDR3 is the sequence as set forth in SEQ ID NO.9 or has at least 80%, 85%, 90%, 95% or 99% identity to the sequence as set forth in SEQ ID NO.9; or
(2) the sequence of the HCDR1 is the sequence as set forth in SEQ ID NO. 16 or has at least 80%, 85%, 90%, 95% or 99% identity to the sequence as set forth in SEQ ID NO. 16, the sequence of the HCDR2 is the sequence as set forth in SEQ ID NO. 17 or has at least 80%, 85%, 90%, 95% or 99% identity to the sequence as set forth in SEQ ID NO. 17, and the sequence of the HCDR3 is the sequence as set forth in SEQ ID NO.9 or has at least 80%, 85%, 90%, 95% or 99% identity to the sequence as set forth in SEQ ID NO.9; or
(3) the sequence of the HCDR1 is the sequence as set forth in SEQ ID NO.22 or has at least 80%, 85%, 90%, 95% or 99% identity to the sequence as set forth in SEQ ID NO.22, the sequence of the HCDR2 is the sequence as set forth in SEQ ID NO.23 or has at least 80%, 85%, 90%, 95% or 99% identity to the sequence as set forth in SEQ ID NO.23, and the sequence of the HCDR3 is the sequence as set forth in SEQ ID NO.9 or has at least 80%, 85%, 90%, 95% or 99% identity to the sequence as set forth in SEQ ID NO.9; or
(4) the sequence of the HCDR1 is the sequence as set forth in SEQ ID NO.28 or has at least 80%, 85%, 90%, 95% or 99% identity to the sequence as set forth in SEQ ID NO.28, the sequence of the HCDR2 is the sequence as set forth in SEQ ID NO. 17 or has at least 80%, 85%, 90%, 95% or 99% identity to the sequence as set forth in SEQ ID NO. 17, and the sequence of the HCDR3 is the sequence as set forth in SEQ ID NO.9 or has at least 80%, 85%, 90%, 95% or 99% identity to the sequence as set forth in SEQ ID NO.9; or
(5) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.8 or has at least 80%, 85%, 90%, 95% or 99% identity to the sequence as set forth in SEQ ID NO.8, and the sequence of the HCDR3 is the sequence as set forth in SEQ ID NO.32 or has at least 80%, 85%, 90%, 95% or 99% identity to the sequence as set forth in SEQ ID NO.32; or
(6) the sequence of the HCDR1 is the sequence as set forth in SEQ ID NO.35 or has at least 80%, 85%, 90%, 95% or 99% identity to the sequence as set forth in SEQ ID NO.35, and the sequence of the HCDR2 is the sequence as set forth in SEQ ID NO. 17 or has at least 80%, 85%, 90%, 95% or 99% identity to the sequence as set forth in SEQ ID NO. 17, and the sequence of the HCDR3 is the sequence as set forth in SEQ ID NO.9 or has at least 80%, 85%, 90%, 95% or 99% identity to the sequence as set forth in SEQ ID NO.9; or
(7) the sequence of the HCDR1 is the sequence as set forth in SEQ ID NO.39 or has at least 80%, 85%, 90%, 95% or 99% identity to the sequence as set forth in SEQ ID NO.39, the sequence of the HCDR2 is the sequence as set forth in SEQ ID NO.40 or has at least 80%, 85%, 90%, 95% or 99% identity to the sequence as set forth in SEQ ID NO.40, and the sequence of the HCDR3 is the sequence as set forth in SEQ ID NO.9 or has at least 80%, 85%, 90%, 95% or 99% identity to the sequence as set forth in SEQ ID NO.9; or
(8) the sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.40 or has at least 80%, 85%, 90%, 95% or 99% identity to the sequence as set forth in SEQ ID NO.40, and the sequence of the HCDR3 is the sequence as set forth in SEQ ID NO.9 or has at least 80%, 85%, 90%, 95% or 99% identity to the sequence as set forth in SEQ ID NO.9; or
(9) The sequence of the HCDR1 is the sequence as set forth in SEQ ID NO.46 or has at least 80%, 85%, 90%, 95% or 99% identity to the sequence as set forth in SEQ ID NO.46, the sequence of the HCDR2 is the sequence as set forth in SEQ ID NO.23 or has at least 80%, 85%, 90%, 95% or 99% identity to the sequence as set forth in SEQ ID NO.23, and the sequence of the HCDR3 is the sequence as set forth in SEQ ID NO.9 or has at least 80%, 85%, 90%, 95% or 99% identity to the sequence as set forth in SEQ ID NO.9; or
(10) The sequence of the HCDR1 is the sequence as set forth in SEQ ID NO.7 or has at least 80%, 85%, 90%, 95% or 99% identity to the sequence as set forth in SEQ ID NO.7, the sequence of the HCDR2 is the sequence as set forth in SEQ ID NO.40 or has at least 80%, 85%, 90%, 95% or 99% identity to the sequence as set forth in SEQ ID NO.40, and the sequence of the HCDR3 is the sequence as set forth in SEQ ID NO.32 or has at least 80%, 85%, 90%, 95% or 99% identity to the sequence as set forth in SEQ ID NO.32;
wherein the sequences of the HCDRs are defined according to the IMGT definition.

10. An anti-βKlotho antibody or an antigen-binding portion thereof, comprising a light chain variable region comprising a light chain CDR1 (LCDR1), a light chain CDR2 (LCDR2) and a light chain CDR3 (LCDR3), wherein:
the sequence of the LCDR1 is the sequence as set forth in SEQ ID NO.10 or has at least 80%, 85%, 90%, 95% or 99% identity to the sequence as set forth in SEQ ID NO.10, the sequence of the LCDR2 is the sequence as set forth in SEQ ID NO.11 or has at least 80%, 85%, 90%, 95% or 99% identity to the sequence as set forth in SEQ ID NO.11, and the sequence of the LCDR3 is the sequence as set forth in SEQ ID NO.6 or has at least 80%, 85%, 90%, 95% or 99% identity to the sequence as set forth in SEQ ID NO.6;
wherein the sequences of the LCDRs are defined according to the IMGT definition.

11. The antibody or antigen-binding portion thereof of any one of claims 1 to 10, wherein:
the anti-βKlotho antibody or antigen-binding portion thereof binds to human βKlotho, preferably specifically binds to human βKlotho; and/or
the anti-βKlotho antibody is an intact antibody, a single chain fragment variable (scFv), a bispecific antibody, or a multispecific antibody; and/or
the antigen-binding portion of the anti-βKlotho antibody is a Fab, Fab', Fv or F(ab')₂; and/or
the anti-βKlotho antibody is a murine antibody, a chimeric antibody, a humanized antibody, or a fully human antibody; and/or
the anti-βKlotho antibody is a monoclonal antibody; and/or
the anti-βKlotho antibody is of IgG1, IgG2 or IgG4 isotype; and/or
the anti-βKlotho antibody is of IgG1/IgG4, IgG2/IgG4 or IgG1/IgG2 chimeric type;
the anti-βKlotho antibody comprises a light chain constant region of kappa subtype or lambda subtype; and/or
the anti-βKlotho antibody comprises a human IgG1 heavy chain constant region and a human kappa light chain constant region; and/or
the anti-βKlotho antibody comprises a wild-type Fc region, or comprises an engineered Fc region such that the antibody has attenuated ADCC and/or CDC activites.

12. The antibody or antigen-binding portion thereof of any one of claims 1 to 11, wherein the antibody has the heavy chain variable region as set forth in SEQ ID NO.49, the light chain variable region as set forth in SEQ ID NO.44, the heavy chain constant region as set forth in SEQ ID NO.50, and the light chain constant region as set forth in SEQ ID NO.51.

13. The antibody or antigen-binding portion thereof of any one of claim 1 to 12 for use in medical therapy.

14. An isolated nucleic acid molecule encoding the anti-βKlotho antibody or antigen-binding portion thereof of any one of claims 1 to 13.

15. A vector comprising the nucleic acid molecule of claim 14.

16. A host cell comprising the nucleic acid molecule of claim 14 or the vector of claim 15.

17. An antibody-drug conjugate comprising the anti-βKlotho antibody or antigen-binding portion thereof of any one of claims 1 to 13 conjugated with a therapeutic agent.

18. A pharmaceutical composition comprising the anti-βKlotho antibody or an antigen-binding portion thereof of any one of claims 1 to 13 or the antibody-drug conjugate of claim 17, and a pharmaceutically acceptable carrier.

19. The pharmaceutical composition of claim 18, wherein the pharmaceutical composition is for the treatment of a βKlotho-mediated or βKlotho-FGFR1c-FGF21-mediated disease, for example, a metabolic disorder, such as type 1 diabetes, type 2 diabetes, dyslipidemia, non-alcoholic steatohepatitis (NASH), non-alcoholic fatty liver disease (NAFLD), cardiovascular disease, metabolic syndrome or obesity.

20. Use of the anti-βKlotho antibody or antigen-binding portion thereof of any one of claims 1 to 13, the nucleic acid molecule of claim 14, the vector of claim 15, the host cell of claim 16, or the antibody-drug conjugate of claim 17 for the manufacture of a medicament for the treatment of a βKlotho-mediated or βKlotho-FGFR1c-FGF21-mediated disease, for example, a metabolic disorder, such as type 1 diabetes, type 2 diabetes, dyslipidemia, non-alcoholic steatohepatitis (NASH), non-alcoholic fatty liver disease (NAFLD), cardiovascular disease, metabolic syndrome or obesity.

21. A method of treating a βKlotho-mediated or βKlotho-FGFR1c-FGF21-mediated disease in a subject, comprising administering to the subject a therapeutically effective amount of an anti-βKlotho antibody or antigen-binding portion thereof of any one of claims 1 to 13, the antibody-drug conjugate of claim 17, or the pharmaceutical composition of claim 18, for example, the disease is a metabolic disorder, such as type 1 diabetes, type 2 diabetes, dyslipidemia, non-alcoholic steatohepatitis (NASH), non-alcoholic fatty liver disease (NAFLD), cardiovascular disease, metabolic syndrome or obesity.

22. A conjugate comprising the anti-βKlotho antibody or an antigen-binding portion thereof of any one of claims 1 to 13 and a detectable label.

23. A fusion protein comprising the anti-βKlotho antibody or antigen-binding portion thereof of any one of claims 1 to 13.
